# EUROPEAN PATENT SPECIFICATION

(11) **EP 3 431 106 B1**
(45) Date of publication and mention of the grant of the patent: **30.12.2020**
(21) Application number: 18182695.9
(22) Date of filing: 20.01.2015
(51) Int. Cl.: A61K 31/4015, A61K 31/437, A61K 9/48, A61P 25/08, A61P 25/06, A61P 25/18, A61P 25/02

(54) **COMBINATIONS COMPRISING POSITIVE ALLOSTERIC MODULATORS OF METABOTROPIC GLUTAMATERGIC RECEPTOR SUBTYPE 2 AND THEIR USE**
KOMBINATIONEN MIT POSITIVEN ALLOSTERISCHEN MODULATOREN DES METABOTROPEN GLUTAMATERGEN REZEPTORS VOM SUBTYP 2 UND DEREN VERWENDUNG
COMBINAISONS COMPRENANT DES MODULATEURS ALLOSTÉRIQUES POSITIFS DE RÉCEPTEURS GLUTAMATERGIQUES MÉTABOTROPES DE SOUS-TYPE 2 ET LEUR UTILISATION

(30) Priority: 21.01.2014 US 201461929795 P; 04.02.2014 EP 14153887; 04.02.2014 EP 14153880; 03.09.2014 EP 14183324; 02.10.2014 EP 14187429; 15.12.2014 US 201462091668 P
(43) Date of publication of application: 23.01.2019
(62) Document number(s) of the earlier application(s) in accordance with Art. 76 EPC: 15700726.1 / 3 096 790
(73) Proprietor: Janssen Pharmaceutica NV, 2340 Beerse (BE)
(72) Inventor: KLEIN, Brian, D., Salt Lake City, Utah UT 84108 (US); LAVREYSEN, Hilde, 2340 Beerse (BE); PYPE, Stefan, Maria, Christiaan, 2340 Beerse (BE); TWYMAN, Roy, E., Raritan, New Jersey NJ 08869 (US); VAN OSSELAER, Nancy, Eulalie, Sylvain, 2340 Beerse (BE); WHITE, H., Steven, Salt Lake City, Utah UT 84108 (US); CEUSTERS, Marc André, 2340 Beerse (BE); CID-NÚÑEZ, José, Maria, 28042 Madrid (ES); TRABANCO-SUÁREZ, Andrés, Avelino, 28042 Madrid (ES); BONE, Roger, Francis, London, W1G DBG (GB)
(74) Representative: Garcia Prieto, Maria

(56) References cited:
- EP-A1- 2 462 990
- WO-A1-01/39779
- WO-A1-2014/078568
- WO-A1-2015/032790
- MYHRER TROND ET AL: "Capacities of metabotropic glutamate modulators in counteracting soman-induced seizures in rats", EUROPEAN JOURNAL OF PHARMACOLOGY, vol. 718, no. 1, 7 September 2013 (2013-09-07), pages 253-260, XP028754545, ISSN: 0014-2999, DOI: 10.1016/J.EJPHAR.2013.08.024
- KLITGAARD H: "ANTIEPILEPTIC DRUG DISCOVERY: LESSONS FROM THE PAST AND FUTURE CHALLENGES", ACTA NEUROLOGICA SCANDINAVICA. SUPPLEMENTUM, MUNKSGAARD, COPENHAGEN, DK, vol. 181, 1 January 2005 (2005-01-01), pages 68-72, XP009078178, ISSN: 0065-1427
- PIAZZINI A ET AL: "Levetiracetam: An improvement of attention and of oral fluency in patients with partial epilepsy", EPILEPSY RESEARCH, ELSEVIER SCIENCE PUBLISHERS, AMSTERDAM, NL, vol. 68, no. 3, 1 March 2006 (2006-03-01), pages 181-188, XP027974358, ISSN: 0920-1211 [retrieved on 2006-03-01]
- Cecilie Johannessen Landmark ET AL: "Modifi cations of Antiepileptic Drugs for Improved Tolerability and Effi cacy", Perspectives in medicinal chemistry , 2008(2), 10 April 2008 (2008-04-10), pages 21-39, XP055113204, Retrieved from the Internet: URL:http://www.ncbi.nlm.nih.gov/pmc/articl es/PMC2746576/pdf/pmc-2008-021.pdf [retrieved on 2014-04-09]
- PRICE M J: "Levetiracetam in the Treatment of Neuropathic Pain: Three Case Studies", CLINICAL JOURNAL OF PAIN, NEW YORK, NY, US, vol. 20, no. 1, 1 January 2004 (2004-01-01), pages 33-36, XP008095827, ISSN: 0749-8047, DOI: 10.1097/00002508-200401000-00007

## Description

### FIELD OF THE INVENTION

The present invention relates to combinations comprising a positive allosteric modulator ("PAM") of metabotropic glutamatergic receptor subtype 2 ("mGluR2") or a pharmaceutically acceptable salt or a solvate thereof, and a synaptic vesicle protein 2A ("SV2A") ligand.

### BACKGROUND OF THE INVENTION

Epilepsy describes a condition in which a person has recurrent seizures due to a chronic, underlying process. Epilepsy refers to a clinical phenomenon rather than a single disease entity, since there are many forms and causes of epilepsy. Using a definition of epilepsy as two or more unprovoked seizures, the incidence of epilepsy is estimated at approximately 0.3 to 0.5 percent in different populations throughout the world, with the prevalence of epilepsy estimated at 5 to 10 people per 1000.

An essential step in the evaluation and management of a patient with a seizure is to determine the type of seizure that has occurred. The main characteristic that distinguishes the different categories of seizures is whether the seizure activity is partial (synonymous with focal) or generalized.

Partial seizures are those in which the seizure activity is restricted to discrete areas of the cerebral cortex. If consciousness is fully preserved during the seizure, the clinical manifestations are considered relatively simple and the seizure is termed a simple-partial seizure. If consciousness is impaired, the seizure is termed a complex-partial seizure. An important additional subgroup comprises those seizures that begin as partial seizures and then spread diffusely throughout the cortex, which are known as partial seizures with secondary generalization.

Generalized seizures involve diffuse regions of the brain simultaneously in a bilaterally symmetric fashion. Absence or petit mal seizures are characterized by sudden, brief lapses of consciousness without loss of postural control. Atypical absence seizures typically include a longer duration in the lapse of consciousness, less abrupt onset and cessation, and more obvious motor signs that may include focal or lateralizing features.

Generalized tonic-clonic or grand mal seizures, the main type of generalized seizures, are characterized by abrupt onset, without warning. The initial phase of the seizure is usually tonic contraction of muscles, impaired respiration, a marked enhancement of sympathetic tone leading to increased heart rate, blood pressure, and pupillary size. After 10-20 s, the tonic phase of the seizure typically evolves into the clonic phase, produced by the superimposition of periods of muscle relaxation on the tonic muscle contraction. The periods of relaxation progressively increase until the end of the ictal phase, which usually lasts no more than 1 min. The postictal phase is characterized by unresponsiveness, muscular flaccidity, and excessive salivation that can cause stridorous breathing and partial airway obstruction. Atonic seizures are characterized by sudden loss of postural muscle tone lasting 1-2 s. Consciousness is briefly impaired, but there is usually no postictal confusion. Myoclonic seizures are characterized by a sudden and brief muscle contraction that may involve one part of the body or the entire body.

The synaptic vesicle protein 2A ("SV2A") has been identified as a broad spectrum anticonvulsant target in models of partial and generalized epilepsy. Studies performed in animal models and human tissue suggest that changes in the expression of SV2A are implicated in epilepsy (for a review see for instance: (a) Mendoza-Torreblanca et al. "Synaptic vesicle protein 2A: basic facts and role in synaptic function" European Journal of Neuroscience 2013, pp. 1-11; (b) Kaminski RM, et al. "Targeting SV2A for Discovery of Antiepileptic Drugs". In: Noebels JL, Avoli M, Rogawski MA, et al., editors. Jasper's Basic Mechanisms of the Epilepsies [Internet]. 4th edition. Bethesda (MD): National Center for Biotechnology Information (US); 2012. Available from: http://www.ncbi.nlm.nih.gov/books/NBK98183/).

The exact role of SV2A remains unclear but studies suggest that changes in the expression of SV2A affect synaptic function (Nowack et al. "Levetiracetam reverses synaptic deficits produced by overexpression of SV2A" PLoS One 2011, Volume 6 (12), e29560). It has also been suggested that SV2A is a key player in exocytosis and is involved in neurotransmission (Crowder et al. "Abnormal neurotransmission in mice lacking synaptic vesicle protein 2A (SV2A)" Proc Nat Acad Sci USA 1999, 96, pp. 15268-15273) and studies in knock-out mice suggest that lack of SV2A results in an imbalance between glutamatergic and GABAergic neurotransmission (Venkatesan et al. "Altered balance between excitatory and inhibitory inputs onto CA pyramidal neurons from SV2A-deficient but not SV2B-deficient mice" J Neurosci Res 2012, 90, pp. 2317-2327). Decreased expression of SV2A may be a consequence of seizure activity and may be involved in the progression of epilepsy (van Vliet et al. "Decreased expression of synaptic vesicle protein 2A, the binding site for levetiracetam, during epileptogenesis and chronic epilepsy" Epilepsia 2009, 50, pp. 422-433; Feng et al. "Down-regulation of synaptic vesicle protein 2A in the anterior temporal neocortex of patients with intractable epilepsy" J Mol Neurosci 2009, 39, pp. 354-359; Toering et al. "Expression patterns of synaptic vesicle protein 2A in focal cortical dysplasia and TSC-cortical tubers" Epilepsia 2009, 50, pp. 1409-1418) and epileptogenesis in patients with brain tumours (de Groot et al. "Expression of synaptic vesicle protein 2A in epilepsy-associated brain tumors and in the peritumoral cortex" Neuro-Oncology 2010, 12, pp. 265-273).

SV2A ligands include levetiracetam (Lynch et al. "The synaptic vesicle protein SV2A is the binding site for the antiepileptic drug levetiracetam" Proc. Natl. Acad. Sci. USA 2004, Vol. 101, pp. 9861-9866), brivaracetam and seletracetam (Kaminski RM, et al. "Targeting SV2A for Discovery of Antiepileptic Drugs". In: Noebels JL, Avoli M, Rogawski MA, et al., editors. Jasper's Basic Mechanisms of the Epilepsies [Internet]. 4th edition. Bethesda (MD): National Center for Biotechnology Information (US); 2012. Available from: http://www.ncbi.nlm.nih.gov/books/NBK98183/; Nowack et al. "Levetiracetam reverses synaptic deficits produced by overexpression of SV2A" PLoSone December 2011, Vol. 6(12), e29560).

Levetiracetam, (-)-(S)-α-ethyl-2-oxo-1-pyrrolidine acetamide or (S)-2-(2-oxopyrrolidin-1-yl)butanamide, 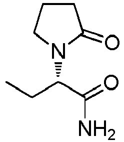 is an antiepileptic drug. It showed no activity in traditional acute models (maximal electroshock and pentylenetetrazol seizure tests) but was found potent in chronic epilepsy models and in genetic models of generalized epilepsy. It has shown a high safety margin compared to other antiepileptic drugs (Klitgaard "Levetiracetam: the preclinical profile of a new class of antiepileptic drugs" Epilepsia 2001, 42 (Supplement 4), pp. 13-18). It is commercialized under the trademark Keppra®, available as tablets, as an oral solution, and as a concentrate made up into a solution for infusion. Keppra® has been approved in Europe as a monotherapy in patients from 16 years of age with newly diagnosed epilepsy, in the treatment of partial-onset seizures (fits) with or without secondary generalization and as an add-on therapy for use with other anti-epileptic drugs in the treatment of partial-onset seizures with or without generalization in patients from 1 month of age; myoclonic seizures in patients from 12 years of age with juvenile myoclonic epilepsy; and primary generalized tonic-clonic seizures in patients from 12 years of age with idiopathic generalized epilepsy (www.ema.europa.eu). Keppra® has also been approved in the USA as an add-on therapy for the treatment of partial onset seizures in patients from 1 month of age; myoclonic seizures in patients 12 years of age and older with juvenile myoclonic epilepsy; and primary generalized tonic-clonic seizures in patients 6 years of age and older with idiopathic generalized epilepsy. Keppra XR®, available as extended-release tablets, has been approved in the USA for the add-on treatment of partial onset seizures in patients 16 years of age and older with epilepsy (http://www.accessdata.fda.gov/scripts/cder/drugsatfda/index.cfm).

Brivaracetam, the 4-n-propyl analog of levetiracetam, (2S)-2-[(4R)-oxo-4-propyl-pyrrolidin-1-yl]butanamide, 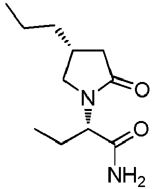 is in clinical trials and investigated as monotherapy in partial onset seizures and post-herpetic neuralgia and as add-on therapy in refractory partial onset seizures, Unverricht-Lundborg disease in adolescents and adults and in photosensitive epilepsy (www.clinicaltrials.gov).

Seletracetam, (2S)-2-[(4S)-4-(2,2,-difluorovinyl)-2-oxo-pyrrolidin-1-yl]butanamide, 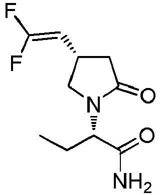 has been tested in clinical trials.

Processes for the preparation of the three compounds are known in the literature. For instance, processes for making Levetiracetam are disclosed for instance, in EP 0 162 036 and in GB 2 225 322. A process for the preparation of Brivaracetam is disclosed for instance in WO 01/62726. A process for the preparation of Seletracetam is known for instance from WO2005/121082. Alternative processes for making the three compounds are disclosed in EP1806339.

Antiepileptic drugs have found usefulness in neurological and psychiatric disorders, including neuropathic pain, migraine, essential tremor and in anxiety, schizophrenia and bipolar disorder (Landmarck "Antiepileptic drugs in non-epilepsy disorders. Relations between mechanisms of action and clinical efficacy" CNS Drugs 2008, Vol. 22(1), pp. 27-47; Calabresi et al. "Antiepileptic drugs in migraine: from clinical aspects to cellular mechanisms" Trends in Pharmacological Sciences 2007, Vol. 28(4), pp. 188-195; Rogawski and Löscher "The neurobiology of antiepileptic drugs for the treatment of nonepileptic conditions" Nat Med 2004, Vol. 10, pp. 685-692).

Levetiracetam has been found effective or potentially effective in a wide-spectrum of neuropsychiatric disorders including mood disorders (Muralidharan and Bhagwagar "Potential of levetiracetam in mood disorders: a preliminary review" CNS Drugs 2006, Vol. 20, pp. 969-979; Mula et al. "The role of anticonvulsant drugs in anxiety disorders: a critical review of the evidence" J Clin Pshycopharmacol 2007, Vol. 27, pp. 263-272), anxiety disorders (Kinrys et al. "Levetiracetam as adjunctive therapy for refractory anxiety disorders" J Clin Psychiatry 2007, Vol. 68, pp. 1010-1013; Zhang et al. "Levetiracetam in social phobia: a placebo controlled pilot study" J Psychopharmacol 2005, Vol. 19, pp. 551-553; Kinrys et al. "Levetiracetam for treatment-refractory posttraumatic stress disorder" J Clin Psychiatry 2006, Vol. 67, pp. 211-214), pain (Enggaard et al. "Specific effect of levetiracetam in experimental human pain models" Eur J Pain 2006, Vol. 10, pp. 193-198; Dunteman "Levetiracetam as an adjunctive analgesic in neoplastic plexopathies: case series and commentary" J Pain Palliative Care Pharmacother 2005, Vol. 19, pp. 35-43; Price "Levetiracetam in the treatment of neuropathic pain: three case studies" Clin J Pain 2004, Vol. 20, pp. 33-36), movement disorders (Bushara et al. "The effect of levetiracetam on essential tremor" Neurology 2005, Vol. 64, pp. 1078-1080; McGavin et al "Levetiracetam as a treatment for tardive dyskinesia: a case report" Neurology 2003, Vol. 61, pp. 419; Woods et al. "Effects of levetiracetam on tardive dyskinesia: a randomized, double-blind, placebo-controlled study" J Clin Psychiatry 2008, Vol. 69, pp. 546-554; Zivkovic et al. "Treatment of tardive dyskinesia with levetiracetam in a transplant patient" Acta Neurol Scand 2008, Vol. 117, pp. 351-353; Striano et al. "Dramatic response to levetiracetam in post-ischaemic Holmes' tremor" J Neurol Neurosurg Psychiatry 2007, Vol. 78, pp. 438-439) and it is suspected to show potentially beneficial effects in cognitive functioning (Piazzini et al. "Levetiracetam: An improvement of attention and of oral fluency in patients with partial epilepsy" Epilepsy Research 2006, Vol. 68, pp. 181-188; de Groot et al. "Levetiracetam improves verbal memory in high-grade glioma patients" Neuro-oncology 2013, Vol. 15(2), pp. 216-223; Bakker et al. "Reduction of hippocampal hyperactivity improves cognition in amnestic mild cognitive impairment" Neuron 2012, Vol. 74, pp. 467-474; for a review: Eddy et al. "The cognitive impact of antiepileptic drugs" Ther Adv Neurol Disord 2011, Vol. 4(6), pp. 385-407 and references cited therein; Wheless "Levetiracetam in the treatment of childhood epilepsy" Neuropsychiatric Disease and Treatment 2007, Vol. 3(4), pp. 409-421), and behavioral symptoms in dementia (Dolder and Nealy "The efficacy and safety of newer anticonvulsants in patients with dementia" Drugs Aging 2012, Vol. 29(8), pp. 627-637). Animal data and some preliminary clinical trials suggest that levetiracetam may have potential for restraining post-traumatic epilepsy, such as those caused by status epilepticus, traumatic brain injury and ischemic stroke, and it appears to have neuroprotective effects. The potential of levetiracetam in easing epileptogenesis or cognitive dysfunction remains to be ascertained by conclusive animal and clinical studies (for reviews: Löscher and Brandt "Prevention or modification of epileptogenesis after brain insults: experimental approaches and translational research" Pharmacol Rev 2010, Vol. 62, 668-700; Shetty "Prospects of levetiracetam as a neuroprotective drug against status epilepticus, traumatic brain injury and stroke" Front. Neur.2013, 4:172. Doi: 10.3389/fneur.2013.00172) as it has displayed antiepileptogenic activity in the kindling model in mice and rats. It has also been suggested that levetiracetam inhibits glutamate release (Lee et al. "Levetiracetam inhibits glutamate transmission through presynaptic P/Q-type calcium channels on the granule cells of the dentate gyrus" British Journal of Pharmacology 2009, Vol. 158, pp. 1753-1762).

Seletracetam and Brivaracetam, have been found to reduce the severity of dystonia in the dt^{sz} mutant hamster model and may be helpful in some patients suffering from dyskinetic and dystonic movement disorders (Hamann et al. "Brivaracetam and seletracetam, two new SV2A ligands, improve paroxysmal dystonia in the dtsz mutant hamster" European Journal of Pharmacology 2008, Vol. 601, pp. 99-102).

Positive allosteric modulators of mGluR2 have emerged recently as promising novel therapeutic approaches for the treatment of several CNS disorders, including epilepsy, and some mGluR2 PAMs are currently undergoing clinical trials for the treatment of schizophrenia, and anxiety-depression (www.clinicaltrials.gov, see for instance: JNJ-40411813/ADX71149 by Addex Therapeutics and Janssen Pharmaceuticals, Inc.). The initial suggestion that drugs that dampen glutamatergic transmission may be efficacious in the treatment of epilepsy came from acute non-clinical studies with mixed mGlu2/3 receptor agonists (Moldrich et al. "Glutamate metabotropic receptors as targets for drug therapy in epilepsy" Eur J Pharmacol. 2003, Vol. 476, pp. 3-16). LY379268 and LY389795, two mGlu2/3 receptor agonists, were found ineffective in blocking MES seizures up to doses producing motor impairment but were found effective in the 6 Hz model in a dose-dependent manner (Barton et al. "Comparison of the effect of glutamate receptor modulators in the 6 Hz and maximal electroshock seizure models" Epilepsy Research 2003, Vol. 56, pp. 17-26). Continued administration of an mGlu2/3 agonist paradoxically induced seizure activity in long-term toxicology studies (Dunayevich et al. "Efficacy and tolerability of an mGlu2/3 agonist in the treatment of generalized anxiety disorder" Neuropsychopharmacology. 2008, Vol. 33(7), pp. 1603-10). This paradoxical effect may be related to agonist-induced changes in the sensitivity of the receptor system (tachyphylaxis), but has not been reported however in preclinical models of epilepsy. Positive allosteric modulators, in contrast, modulate ongoing neurotransmission but are not directly stimulatory, thereby reducing the risk for tachyphylaxis.

Prior to seizure activity, increases in extracellular glutamate are measured in human hippocampus and the increase is sustained during epileptogenic activity (During and Spencer "Extracellular hippocampal glutamate and spontaneous seizure in the conscious human brain" Lancet 1993, Vol. 341(8861), pp. 1607-10), thus lending support to the idea that a reduction in glutamate levels may be of benefit in the treatment of epilepsy. In fact, during seizure activity glutamate levels increase to potentially neurotoxic levels. Seizure activity results in progressive structural damage in human brain inducing further abnormalities in glutamate metabolism (Petroff et al. "Glutamate-glutamine cycling in the epileptic human hippocampus" Epilepsia 2002, Vol. 43(7), pp. 703-10). Thus, an mGluR2 positive allosteric modulator or an mGluR2 orthosteric agonist may be expected to protect against seizure-induced neuronal damage.

WO2009/033704 and WO2010/130424 disclose mGluR2 positive allosteric modulators, uses thereof and processes for synthesizing the compounds. WO1997/18199 and WO2003/104217 disclose excitatory amino acid receptor modulator compounds that later were shown to have mGlu2/3 orthosteric agonist activity (see for example Rorick-Kehn et al. (2007) The Journal of Pharmacology and Experimental therapeutics Vol. 321, No. 1, pp. 308-317), further scientific and patent literature disclose additional examples of compounds having mGlu2/3 orthosteric agonist activity, and WO2008/150233 discloses compounds with mGluR2 allosteric activator activity.

Myhrer, T. et al. "Capacities of metabotropic glutamate modulators in counteracting soman-induced seizures in rats" Eur. J. Pharmacol. 718 (2013) 253-260 discloses the effect of DCG-IV combinations against soman-induced seizures in rats. While combination of HI-6 and procyclidine with DCG-IV showed antidotal efficacy, combination of HI-6 and levetiracetam with DCG-IV did not terminate seizures or preserve lives, and the observed antidotal efficacy was lower.

Klitgaard, H. "Antiepileptic drug discovery: lessons from the past and future challenges" Acta Neurol Scand 2005: 112 (Suppl. 181): 68-72 provides a brief historical background into antiepileptic drug research, and describes the discovery of levetiracetam and subsequent identification of brivaracetam and seletracetam, having a unique anticonvulsant activity profile, and potentially better safety profile compared to existing therapies.

Piazzini et al. "Levetiracetam: An improvement of attention and of oral fluency in patients with partial epilepsy" Epilepsy Research 2006, Vol. 68, pp. 181-188 discloses preliminary results showing a significant cognitive improvement in attention and oral fluency in patients diagnosed with drug resistant partial epilepsy receiving levetiracetam as add-on to therapy compared to controls.

Johannessen Landmark, C and Johannessen, S.I. "Modifications of Antiepileptic Drugs for Improved Tolerability and Efficacy" Perspect Medicin Chem 2008:2 21-39 discloses a review on the design and marketed drugs and investigational products in development in 2008, including levetiracetam.

Price, M.J. "Levetiracetam in the treatment of neuropathic pain: three case studies" Clin J Pain 2004, Vol. 20, pp. 33-36 describes three case studies of patients presenting with neuropathic pain responding favourably to treatment with levetiracetam, with no important adverse events.

WO 01/39779 (UCB, S.A.) discloses the use of levetiracetam in disorders including migraine, bipolar disorders, and chronic or neuropathic pain alone or in combination with compounds inducing neural inhibition mediated by GABA_{A} receptors.

EP 2 462 990 (UCB Pharma GmbH) describes a pharmaceutical composition comprising lacosamide and levetiracetam for the prevention, alleviation and/or treatment of epileptic seizures.

WO 2014/078568 (Univ Johns Hopkins) relates in broad terms to methods for treating schizophrenia or bipolar disorder by administering a combination of a synaptic vesicle protein 2A inhibitor and an antipsychotic. The examples disclose however, levetiracetam, brivaracetam or valproate in studies related to cognition (spatial learning and memory retention, trials in patients with amnestic mild cognitive impairment).

WO2015/032790 (Janssen Pharmaceutica NV) describes 1,2,4-triazolo[4,3-a]pyridine compounds as positive allosteric modulators of the metabotropic glutamate receptor subtype 2.

Currently available anti-epileptic drugs do not solely affect glutamatergic transmission. Their mechanism of action is generally conceptualized as altering the balance between excitatory (glutamate-mediated) and inhibitory (GABA-mediated) transmission (Johannessen Landmark "Antiepileptic drugs in non-epilepsy disorders: relations between mechanisms of action and clinical efficacy" CNS Drugs 2008, Vol. 22(1), pp. 27-47).

A significant limiting factor in the use of SV2A ligands is tolerability and side-effect profile. For example the effective dose of levetiracetam for partial onset seizures is dosed at 1000 mg, 2000 mg, and 3000 mg, given as twice-daily. The side effects reported for levetiracetam include aggressive or angry behavior, anxiety, change in personality, chills, cough or hoarseness, crying, depersonalization, diarrhea, dry mouth, euphoria, fever, general feeling of discomfort or illness, headache, hyperventilation, irregular heartbeats, irritability, joint pain, loss of appetite, lower back or side pain, mental depression, muscle aches and pains, nausea, painful or difficult urination, paranoia, quick to react or overreact emotionally, rapidly changing moods, restlessness, shaking, shivering, shortness of breath, sleepiness or unusual drowsiness, sore throat, stuffy or runny nose, sweating, trouble sleeping, unusual tiredness or weakness and vomiting. Thus, there is still a need to provide an effective treatment with a lower effective dose of levetiracetam and a more favourable side effect profile for the treatment of epilepsy and related disorders, not only in the adult but also in the pediatric population.

### BRIEF DESCRIPTION OF THE FIGURES

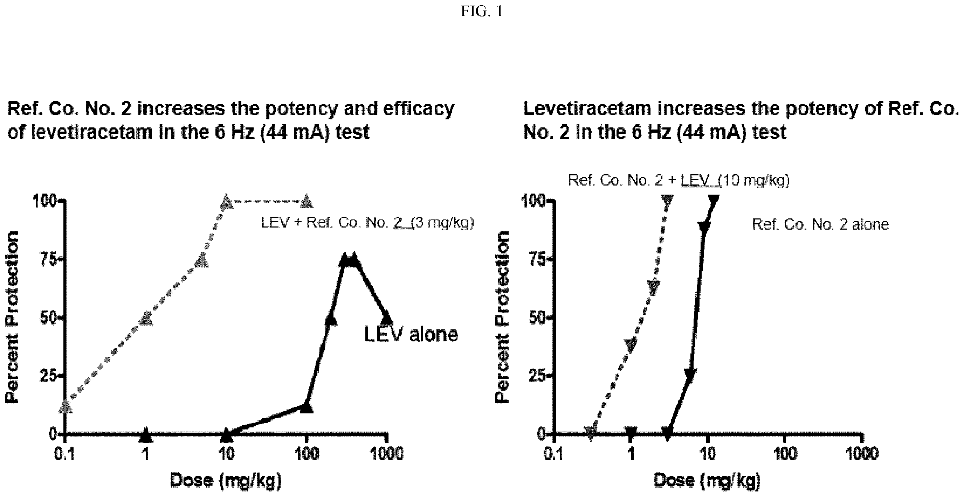

**Figure 1****:** Dose response for the 6Hz 44mA ED₅₀ determination for the reference Co. No. **2** and LEV alone and in combination.

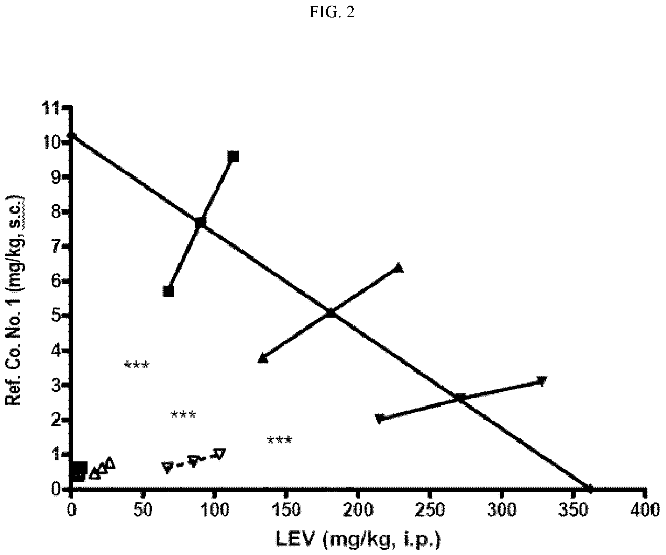

**Figure 2****:** Isobolographic analysis for the combination of reference Co. No. **1** with levetiracetam (LEV) in the 6Hz (44 mA) assay. Initial ED₅₀ values (shown below) were determined for both reference Co. No. **1** and LEV (data points on x- and y-axes; filled diamonds). The theoretical line of additivity connects the calculated ED₅₀ values for the two compounds (solid black line). Theoretical ED₅₀ (+ SEM) for three fixed dose ratio combinations (LEV: reference Co. No. **1**) are plotted: 1:3 - filled squares/solid black line, 1:1 - filled upward triangles/solid black line, and 3:1 - filled downward triangles/solid black line. Experimental treatment doses were initially derived from theoretical values and adjusted according to observed effects. Experimentally-determined ED₅₀ (+ SEM) values for each fixed dose-ratio combination are also shown: 1:3' - open squares/dotted line, 1:1' - open upward triangles/dotted line, and 3:1' - open downward triangles/dotted line. Comparisons between theoretical and experimentally-determined ED₅₀ values were compared using a t-test (***P<0.001). N=8 per group. In Figure 2, the ratio of LEV to reference Co. No. **1** is depicted as follows:

| LEV : reference Co. No. 1 ratio | | |
|---|---|---|
| 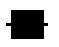 | 1:3 | |
| 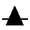 | 1:1 | |
| 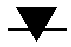 | 3:1 | |
| 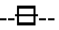 | 1:3' | ED₅₀ (LEV) = 345 mg/kg (211-485) (intraperitoneally, i.p.) |
| 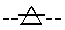 | 1:1' | |
| 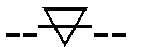 | 3:1' | ED₅₀ (reference Co. No. **1**) = 10.2 mg/kg (3.1-12.4) (s.c.) |

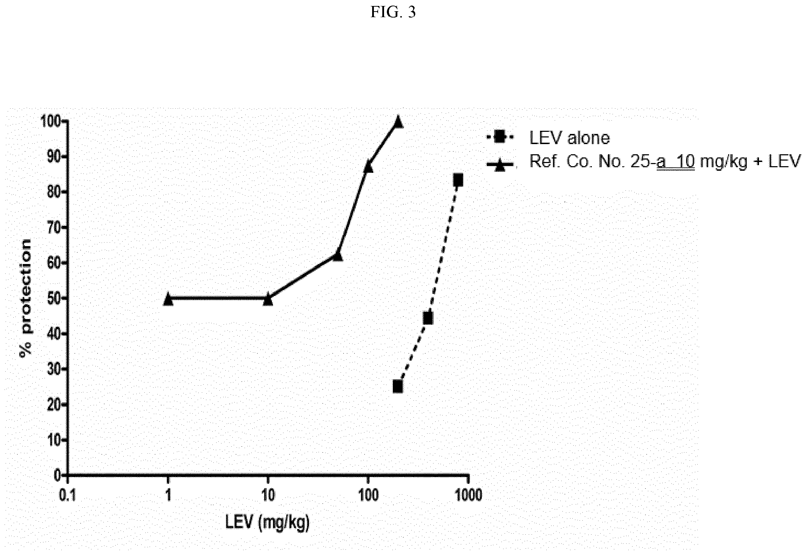

**Figure 3****:** Combination Studies for reference Co. No. **25-a** with levetiracetam (LEV) in the 6Hz Assay (44 mA). At a dose of 10 mg/kg s.c., reference Co. No. **25-a** increases the potency of LEV, leading to an approximate 70-fold shift in the ED₅₀. This suggests a positive pharmacodynamic relationship.

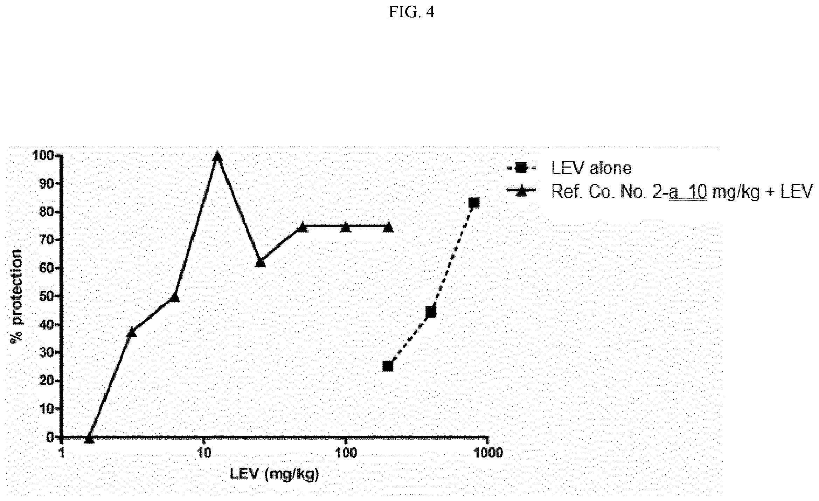

**Figure 4****:** Combination Studies for reference Co. No. 2-a with levetiracetam (LEV) in the 6 Hz Assay (44 mA). At a dose of 10 mg/kg s.c., reference Co. No. **2-a** increases the potency of LEV, leading to an approximate 35-fold shift in the ED₅₀. This suggests a positive pharmacodynamic relationship.

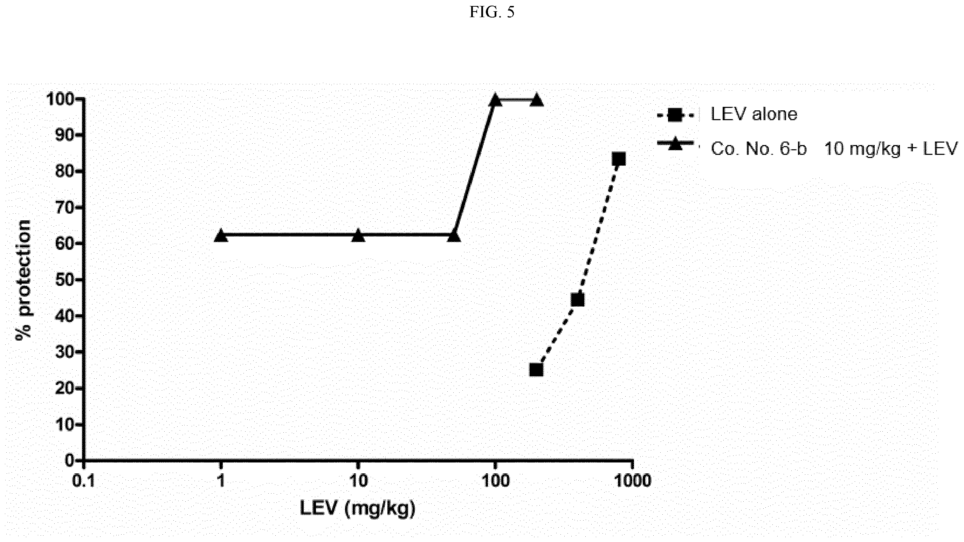

**Figure 5****:** Combination Studies for Co. No. **6-b** with levetiracetam (LEV) in the 6 Hz Assay (44 mA). At a dose of 10 mg/kg p.o., Co. No. **6-b** increases the potency of LEV, leading to an approximate 100-fold shift in the ED₅₀. This suggests a positive pharmacodynamic relationship.

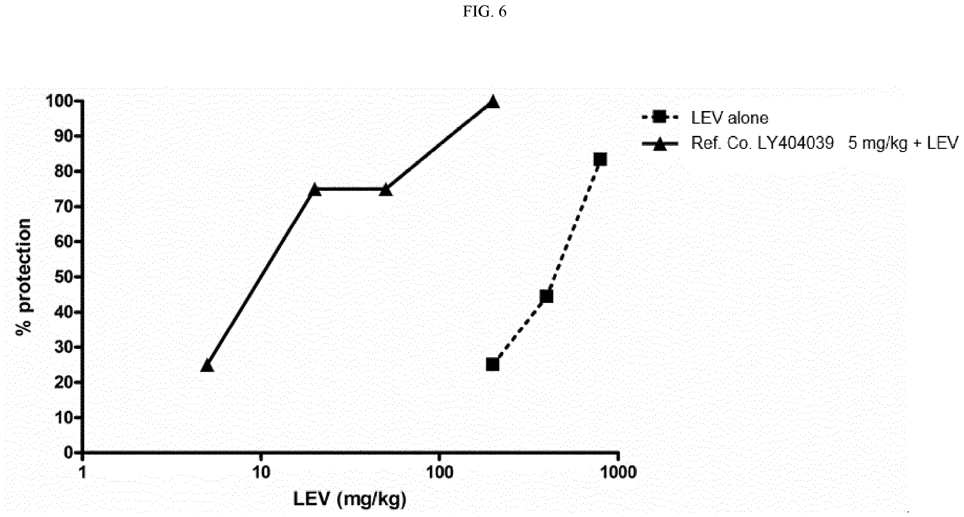

**Figure 6****:** Combination Studies for reference compound LY-404039 with levetiracetam (LEV) in the 6Hz Assay (44mA). At a dose of 5 mg/kg s.c., reference compound LY-404039 increases the potency of LEV, leading to an approximate 27-fold shift in the ED₅₀. This suggests a positive pharmacodynamic relationship.

### DESCRIPTION OF THE INVENTION

The present invention relates to a combination comprising
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 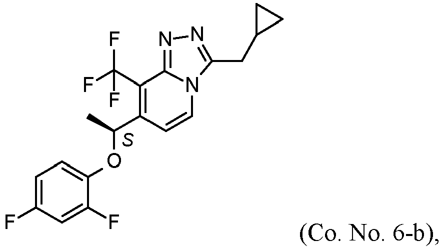 or a pharmaceutically acceptable salt or a solvate thereof.

In a particular embodiment, the invention as described herein relates to a pharmaceutical combination, in particular a pharmaceutical combination product, comprising
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 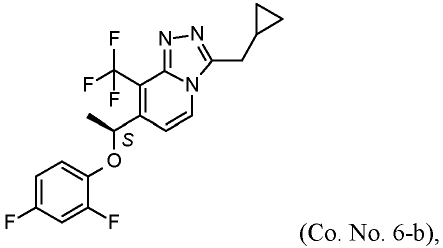 or a pharmaceutically acceptable salt or a solvate thereof; and (c) at least one pharmaceutically acceptable carrier.

In a further embodiment, the invention relates to the combination described herein for use as a medicament.

A further embodiment of this invention relates to the use of the combination described herein for the manufacture of a medicament or a pharmaceutical product for the treatment or prevention of epilepsy and related disorders; neuropathic pain; migraine or resistant headache and bipolar and related disorders.

A further embodiment of this invention relates to the use of the combination described herein for the manufacture of a medicament or a pharmaceutical product for neuroprotection.

A further embodiment of this invention relates to the use of the combination described herein for the manufacture of a medicament or a pharmaceutical product for the prevention of epileptogenesis.

A further embodiment relates to the treatment or prevention of epilepsy and related disorders; neuropathic pain; migraine or resistant headache; and bipolar and related disorders of a subject comprising administering concurrently or sequentially to the subject in need thereof a synaptic vesicle protein 2A ("SV2A") ligand selected from levetiracetam, brivaracetam and seletracetam; and a positive allosteric modulator ("PAM") of metabotropic glutamatergic receptor subtype 2 ("mGluR2") compound selected from 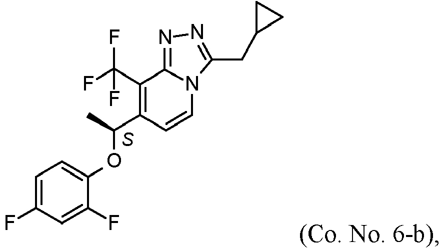 or a pharmaceutically acceptable salt or a solvate thereof, in amounts that would be therapeutically effective when the SV2A ligand and mGluR2 compound are administered together.

A further embodiment relates to a combination as described herein for neuroprotection; or to a combination as described herein for use in neuroprotection.

A further embodiment relates to a combination as described herein for the prevention of epileptogenesis; or to a combination as described herein for use in the prevention of epileptogenesis.

In a further embodiment the invention relates to a fixed dose combination of
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 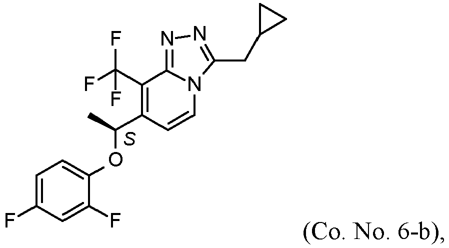 or a pharmaceutically acceptable salt or a solvate thereof,
in amounts that would be therapeutically effective when the SV2A ligand and mGluR2 compound are administered together to patients, for use in treating or preventing epilepsy and related disorders; neuropathic pain; migraine or resistant headache; bipolar and related disorders.

A disclosure relates to a method for the treatment or prevention of epilepsy and related disorders; neuropathic pain; migraine or resistant headache; bipolar and related disorders said method comprising administering a therapeutically effective amount of a combination or a combination product comprising
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 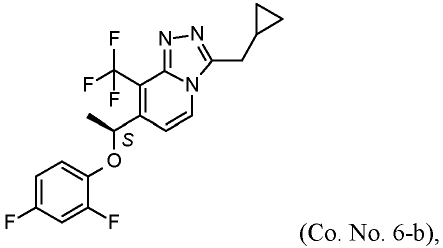 or a pharmaceutically acceptable salt or a solvate thereof,
to a subject in need thereof, such as a warm-blooded animal, in particular a human.

A further disclosure relates to a method of neuroprotection, said method comprising administering a therapeutically effective amount of a combination or a combination product comprising
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 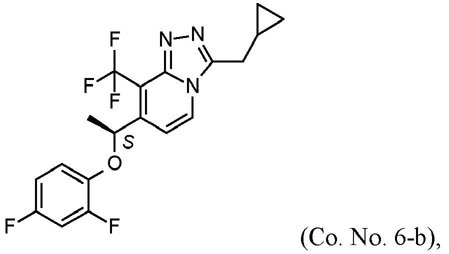 or a pharmaceutically acceptable salt or a solvate thereof,
to a subject in need thereof, such as a warm-blooded animal, in particular a human.

A further disclosure relates to a method of anti-epileptogenesis, said method comprising administering a therapeutically effective amount of a combination or a combination product comprising
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 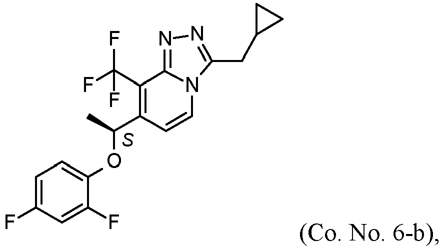 or a pharmaceutically acceptable salt or a solvate thereof,
to a subject in need thereof, such as a warm-blooded animal, in particular a human.

In an additional embodiment, the present invention relates to a pharmaceutical product or a commercial package comprising a combination according to the invention as described herein, in particular together with instructions, for simultaneous, separate or sequential use thereof in the treatment or prevention of epilepsy and related disorders; neuropathic pain; migraine or resistant headache bipolar; and related disorders.

In an additional embodiment, the present invention relates to a pharmaceutical product or a commercial package comprising a combination according to the invention as described herein, in particular together with instructions, for simultaneous, separate or sequential use thereof in neuroprotection.

In an additional embodiment, the present invention relates to a pharmaceutical product or a commercial package comprising a combination according to the invention as described herein, in particular together with instructions, for simultaneous, separate or sequential use thereof in anti-epileptogenesis.

In a further embodiment the invention relates to a combination comprising a quantity which is jointly therapeutically effective against epilepsy and related disorders; neuropathic pain; migraine or resistant headache; bipolar and related disorders; of
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 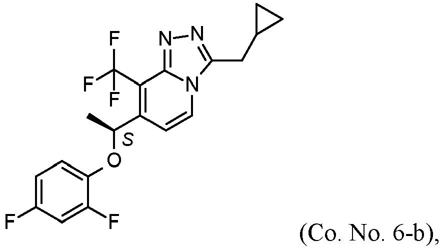 or a pharmaceutically acceptable salt or a solvate thereof, and at least one pharmaceutically acceptable carrier.

In a further embodiment the invention relates to a combination comprising a quantity which is jointly therapeutically effective as neuroprotectant, of
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 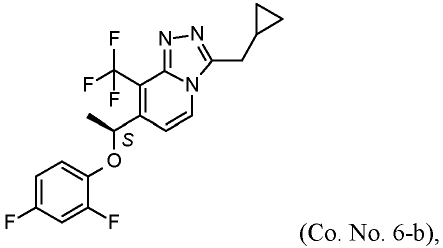 or a pharmaceutically acceptable salt or a solvate thereof, and at least one pharmaceutically acceptable carrier.

In a further embodiment the invention relates to a combination comprising a quantity which is jointly therapeutically effective in the prevention of epileptogenesis, of
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 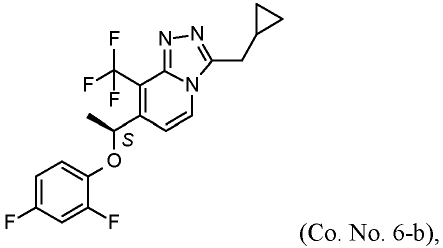 or a pharmaceutically acceptable salt or a solvate thereof, and at least one pharmaceutically acceptable carrier.

In a further embodiment, the invention relates to the use of
(a) a synaptic vesicle protein 2A ("SV2A") ligand selected from levetiracetam, brivaracetam and seletracetam; and
(b) a positive allosteric modulator ("PAM") of metabotropic glutamatergic receptor subtype 2 ("mGluR2") compound selected from 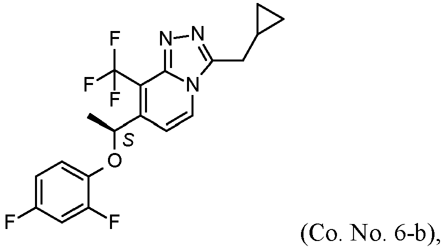 or a pharmaceutically acceptable salt or a solvate thereof,
for the preparation of a combination product according to the present invention.

The (b) component of the combination of the invention is in general referred to herein as "mGluR2 compound" or "mGluR2 PAM compound", or "positive allosteric modulator of mGluR2 compound" meaning that the compound has mainly activity at the metabotropic glutamatergic receptor subtype 2, and is in particular a positive allosteric modulator (PAM) of metabotropic glutamatergic receptor subtype 2. A skilled person will be familiar with the large homology of mGluR2 and mGluR3, due to which some mGluR2 orthosteric agonists also display activity as mGluR3 orthosteric agonists. Such is the case for example, of (-)-(1R,4S,5S,6S)-4-amino-2-sulfonylbicyclo[3.1.0]-hexane-4,6-dicarboxylic acid (also known as LY-404,039 [CAS 635318-11-5]), with a Kᵢ = 149 nM (mGlu2 receptor) and Kᵢ = 92 nM (mGlu3 receptor), 100-fold selectivity for mGlu2 and mGlu3 over mGlu4a, -6, -7a, and -8a, and no activity at mGlu1a and mGlu5a (Rorick-Kehn et al. (2007) The Journal of Pharmacology and Experimental Therapeutics Vol. 321, No. 1, pp. 308-317). The term "mGluR2 compound" or "mGluR2 PAM compound", or "positive allosteric modulator of mGluR2 compound" does therefore not exclude compounds displaying some other additional minor activity in vitro or in vivo.

Reference mGluR2 PAM compounds of the combination of the disclosure are in particular selected from those disclosed in WO2010/130424,and may be prepared according to the processes described therein. Particular reference compounds include

| | |
|---|---|
| 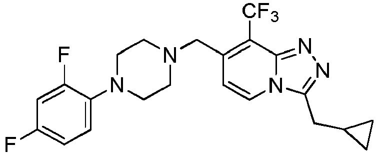 | 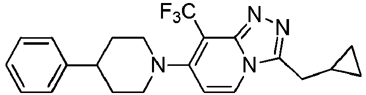 |
| **Ref. Co. No. 1;** or a hydrochloride salt thereof (Ref. **Co. No. 1a**) | **Ref. Co. No. 2;** or a hydrochloride salt thereof (.HCl) (Ref. **Co. No. 2a**) |
| 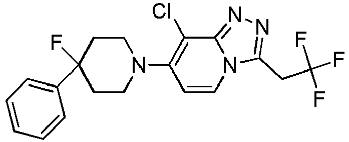 | |
| **Ref. Co. No. 11.** | |

Additional mGluR2 PAM reference compounds of the combination of the disclosure are also in particular selected from those disclosed in WO2009/033704, and may be prepared according to the processes described therein. Particular compounds include 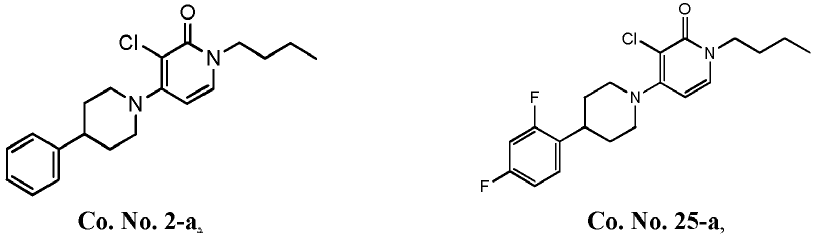 or a pharmaceutically acceptable salt or a solvate thereof.

The mGluR2 PAM compound of the combination of the invention is in particular selected from those disclosed in PCT/EP2014/068676. The particular compound is 3-(Cyclopropylmethyl)-7-[(1S)-1-(2,4-difluorophenoxy)ethyl]-8-(trifluoromethyl)[1,2,4]triazolo[4,3-a]pyridine; including, the pharmaceutically acceptable salts and the solvates thereof.

In an additional embodiment, the compound may be selected from 3-(Cyclopropylmethyl)-7-[(1S)-1-(2,4-difluorophenoxy)ethyl]-8-(trifluoromethyl)[1,2,4]triazolo[4,3-a]pyridine hydrochloride salt.

The names of the compounds of the present invention were generated according to the nomenclature rules agreed upon by the Chemical Abstracts Service (C.A.S.) using Advanced Chemical Development, Inc., software (ACD/Name product version 10.01.0.14105, October 2006).

As used herein, unless otherwise noted, the term **"antiepileptic agent"** and the abbreviation "AED" will be used interchangeably with the term **"anticonvulsant agent",** and as used herein, refer to an agent capable of treating, inhibiting or preventing seizure activity or ictogenesis when the agent is administered to a subject or patient.
As used herein, unless otherwise noted, the term **"synaptic vesicle protein 2A ligand"** and the abbreviation **"SV2A ligand"** will be used interchangeably. Examples of SV2A ligands include, the compounds included in the publications GB 1,039,113; GB 1,309,692; EP 1 262 036; EP 1 806 339; WO 2001/062726; US 2002/094787; WO 2004/087658; WO 2005/121082; WO 2005/054188; WO 2006/128692; WO 2006/128693; WO 2007/065595; WO 2008/132139, and WO 2008/132142; WO 2011/047860; WO 2012/143116; and WO 2012/143117. Suitable particular examples of SV2A ligands include: levetiracetam, brivaracetam and seletracetam.

In a particular embodiment, the SV2A ligand is levetiracetam.

In a particular embodiment, the SV2A ligand is brivaracetam.

Processes for the preparation of the above SV2A ligands are known from the literature and described for instance in EP 1 806 339; in EP 0 162 036 and in GB 2 225 322 (levetiracetam); in WO 01/62726 (brivaracetam); and in WO 2005/121082 (seletracetam).

In an additional embodiment, the combination according to the invention comprises (a) a pharmaceutically effective amount of levetiracetam or brivaracetam; and (b) a pharmaceutically effective amount of 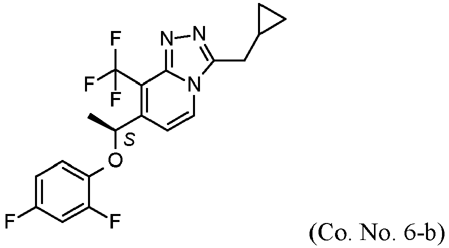 or a pharmaceutically acceptable salt thereof, in particular the hydrochloride salt thereof, or a solvate thereof.

In an additional embodiment, the pharmaceutical composition according to the invention comprises (a) a pharmaceutically effective amount of levetiracetam or brivaracetam; and (b) a pharmaceutically effective amount of 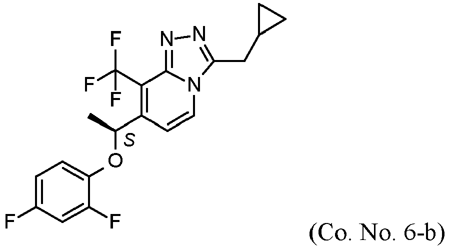 or a pharmaceutically acceptable salt thereof, in particular the hydrochloride salt thereof, or a solvate thereof.

The combination product of the present invention, in particular, the pharmaceutical composition according to the invention, is especially appropriate for the treatment of epilepsy and related disorders.

It will be appreciated that some of the mGluR2 compound, in particular the mGluR2 PAM compound of the invention and its pharmaceutically acceptable addition salts and solvates thereof may contain one or more centres of chirality and exist as stereoisomeric forms.

The term **"compound of the invention"** as used herein, is meant to include compound 6-b as disclosed herein, and the salts and solvates thereof.

As used herein, any chemical formula with bonds shown only as solid lines and not as solid wedged or hashed wedged bonds, or otherwise indicated as having a particular configuration (e.g. R, S) around one or more atoms, contemplates each possible stereoisomer, or mixture of two or more stereoisomers.

Hereinbefore and hereinafter, the absolute configuration is specified according to the Cahn-Ingold-Prelog system. The configuration at an asymmetric atom is specified by either R or S.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a mGluR2 compound is for instance specified as (R), this means that the compound is substantially free of the (S) isomer; when a mGluR2 compound is for instance specified as E, this means that the compound is substantially free of the Z isomer; when a mGluR2 compound is for instance specified as cis, this means that the compound is substantially free of the trans isomer.

For use in medicine, the salts of the compound of this invention refer to non-toxic **"pharmaceutically acceptable salts"** (salts of the compounds of the present invention wherein the counterion is pharmaceutically acceptable). Other salts may, however, be useful in the preparation or purification of compounds according to this invention or of their pharmaceutically acceptable salts, and may encompass acids and bases which are non-pharmaceutically acceptable. All salts, whether pharmaceutically acceptable or not, are included within the ambit of the present invention.

The pharmaceutically acceptable acid and base addition salts as mentioned hereinabove or hereinafter are meant to comprise the therapeutically active non-toxic acid and base addition salt forms which the compounds of the invention are able to form. Suitable pharmaceutically acceptable salts of the compounds include acid addition salts which may, for example, be formed by mixing a solution of the compound with a solution of a pharmaceutically acceptable acid such as for example, inorganic acids such as hydrohalic acids, e.g. hydrochloric or hydrobromic acid, sulfuric, nitric or phosphoric acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic and pamoic acids. Conversely, said salt forms can be converted by treatment with an appropriate base into the free base form. Furthermore, where the compounds of the invention carry an acidic moiety, suitable pharmaceutically acceptable salts thereof may include organic and inorganic bases. Appropriate base salt forms comprise, for example, the ammonium salts, the alkali and alkaline earth metal salts, e.g. the lithium, sodium, potassium, magnesium and calcium salts, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine and lysine. Conversely, the salt form can be converted by treatment with acid into the free acid form.

The term **"solvate"** comprises the solvent addition forms as well as the salts thereof, which the compound 6-b is able to form. Examples of such solvent addition forms are e.g. hydrates and alcoholates.

### Preparation of the compound

The compound according to the invention can generally be prepared by a succession of steps, each of which is known to the skilled person. In particular, the compound can be prepared according to the following synthesis methods.

The compound may be synthesized in the form of racemic mixtures of enantiomers which can be separated from one another following art-known resolution procedures. The racemic compound may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compound involves liquid chromatography or supercritical fluid chromatography (SFC) using a chiral stationary phase. Said pure stereochemically isomeric forms may also be derived from the corresponding pure stereochemically isomeric forms of the appropriate starting materials, provided that the reaction occurs stereospecifically.

As used herein, the term **"composition"** is intended to encompass a product comprising the specified ingredients in the specified amounts, as well as any product which results, directly or indirectly, from combinations of the specified ingredients in the specified amounts.

As used herein, the term **"subject"** refers to an animal, preferably a mammal, most preferably a human adult, child or infant, who is or has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of one or more of the symptoms of the disease or disorder being treated; and/or reduction of the severity of one or more of the symptoms of the disease being treated.

The combination of (a) SV2A ligand and (b) positive allosteric modulator ("PAM") of metabotropic glutamatergic receptor subtype 2 ("mGluR2") or a pharmaceutically acceptable salt or a solvate thereof, whether the compounds (a) and (b) are given simultaneously, separately or sequentially, may be beneficial compared to the effect of the compounds (a) or (b) administered alone. In particular, there may be at least one beneficial effect, e.g. a mutual enhancement of the effect of the compounds (a) and (b), a more than additive effect, in particular a synergic effect; additional advantageous effects, include for example, a significantly reduced effective dose for the combination of (a) and (b); a further therapeutic effect not observed for any of the compounds (a) or (b) alone, a more beneficial side effect profile, or a combined therapeutic effect in a non-effective dosage of one or both of (a) and (b).

Wherein the present invention is directed to co-therapy or combination therapy, comprising administration of (a) synaptic vesicle protein 2A ("SV2A") ligand; and (b) compound 6-b as defined herein, pharmaceutically or therapeutically effective amount shall mean that amount of the combination of agents taken together so that the combined effect elicits the desired biological or medicinal response. For example, the therapeutically effective amount of co-therapy comprising administration of (a) a SV2A ligand as defined herein and (b) compound 6-b as defined herein would be the amount of the (a) a SV2A ligand as defined herein and the amount of (b) a compound 6-b that when taken together or sequentially have a combined effect that is therapeutically effective. Further, it will be recognized by one skilled in the art that in the case of co-therapy with a therapeutically effective amount, as in the example above, the amount of compound 6-b, and/or the amount of the suitable SV2A ligand individually may or may not be therapeutically effective.

The present invention provides methods of prevention or treatment comprising administering to a subject in need thereof, co-therapy with a therapeutically effective amount of a SV2A ligand and a therapeutically effective amount of compound 6-b, as described herein. In order to accomplish this objective the compounds or compositions of this invention must be used in the correct therapeutically effective amount or dose, as described below.

Optimal dosages and schedules to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that a therapeutically effective dosage of the compounds of the present invention can include repeated doses within a prolonged treatment regimen that will yield clinically significant results.

The amounts of the compound 6-b, in the combinations of the invention that are administered on a daily basis may vary from about 0.01 to about 2000 mg. Examples of daily amounts of compound 6-b are 0.01, 0.05, 0.1, 0.5, 1.0, 2.5, 5.0, 10.0, 15.0, 25.0, 50.0, 100, 150, 200, 250, 300, 400, 500, 750 and 1000 milligrams for the symptomatic adjustment of the dosage to the patient to be treated. An effective amount of the drug is ordinarily supplied at a dosage level of from about 0.01 mg/kg to about 150.0 mg/kg of body weight per day or any range therein. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1, 2, 3 or 4 times per day. The amounts of SV2A ligand that are administered on a daily basis may vary from about 0.01 to about 7000 mg, preferably will be between 250 and 5000 mg and more preferably will be between 500 and 3000 mg. Examples of daily amount of the SV2A ligand are 2.5, 5.0, 10.0, 15.0, 25.0, 50.0, 100, 150, 200, 250, 500, 750, 1000, 1500 and 3000 milligrams for the symptomatic adjustment of the dosage of the patient to be treated. An effective amount of the drug is ordinarily supplied at a dosage level of from about 0.01 mg/kg to about 150.0 mg/kg of body weight per day or any range therein. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1, 2, 3 or 4 times per day. All amounts mentioned in this and the following paragraphs refer to the free form (i.e. non-salt form). The above values represent free-form equivalents, i.e. quantities as if the free form would be administered. If salts are administered the amounts need to be calculated in function of the molecular weight ratio between the salt and the free form.

The above mentioned daily doses are calculated for an average body weight of about 70 kg and should be recalculated in case of paediatric applications, or when used with patients with a substantially diverting body weight.

The dosages may be presented as one, two, three or four or more sub-doses administered at appropriate intervals throughout the day. The dosage used preferably corresponds to the daily amount of compound 6-b, or of the SV2A ligand, mentioned above, or a sub-dose thereof, such as 1/2, 1/3, 1/4 thereof. A dosage form may contain the compound 6-b, or the SV2A ligand or both together, in an amount equal to the ranges or quantities mentioned in the previous paragraphs, for example a dosage form may contain 10 mg, 25 mg, 50 mg, 100 mg, 150 mg, or 200 mg of compound 6-b, 10 mg, 25 mg, 50 mg, 100 mg or 250 mg, of SV2A ligand, either in separate formulations or in a combined formulation. In one embodiment, compound 6-b, is administered once daily (q.d.), in particular as one dose per day, and the SV2A ligand is administered once or twice daily (q.d. or b.i.d.), in particular as one or as two doses per day. In the instance where both compounds are to be administered once daily, this can be accomplished by administering two separate doses, one with the compound 6-b, one with the SV2A ligand, or by administering a combined dose containing the compound 6-b, and SV2A ligand.

The combinations of the invention may be administered once, twice, three, four, or if desired, multiple times daily. In one embodiment, the combination is administered once daily. In another embodiment, the combination is administered twice daily, or three times per day. Administration of dosages may be by separate dosage forms, i.e. dosage forms only containing compound 6-b, or only SV2A ligand; or by combined dosage forms containing active ingredients compound 6-b, and SV2A ligand. Also, a mix of using a combined dosage form and separate dosage forms can be used. Dosage forms that can be administered are described hereinafter, oral dosage forms, in particular tablets or capsules being preferred.

Active ingredients may be formulated in pharmaceutical compositions either separately or as a combined pharmaceutical composition. In the latter instance, there is provided a pharmaceutical composition comprising a therapeutically effective amount of the compound 6-b, or a pharmaceutically acceptable salt thereof, and the SV2A ligand, the foregoing being as specified herein, and a pharmaceutically acceptable carrier.

In a further aspect, this invention relates to a process for preparing a pharmaceutical composition as specified herein, which comprises intimately mixing a pharmaceutically acceptable carrier with a therapeutically effective amount of the compound 6-b, or a pharmaceutically acceptable salt or a solvate thereof, and a therapeutically effective amount of at least one SV2A ligand.

The combinations provided herein may also be formulated as a combined preparation for simultaneous, separate or sequential use in the prevention or treatment of epilepsy and related disorders; neuropathic pain; migraine or resistant headache; bipolar and related disorders; in neuroprotection; or in the prevention of epileptogenesis. In such a case, the compound 6-b, is formulated in a pharmaceutical composition containing other pharmaceutically acceptable excipients, and the SV2A ligand is formulated separately in a pharmaceutical composition containing other pharmaceutically acceptable excipients. Conveniently, these separate pharmaceutical compositions can be part of a kit for simultaneous, separate, or sequential use.

The individual components of the combination of the present invention can be administered simultaneously or separately at different times during the course of therapy or concurrently in divided or single combination forms.

Therefore, compound 6-b, and the SV2A ligand, individually or combined, may be formulated into various pharmaceutical compositions suitable for administration purposes. In these, a therapeutically effective amount of the particular compound, or of both two compounds, is combined with a pharmaceutically acceptable carrier, which carrier may take a wide variety of forms depending on the form of preparation desired for administration. Pharmaceutical compositions may be prepared as medicaments to be administered orally, parenterally (including subcutaneously (s.c.), intramuscularly (i.m.), and intravenously (i.v.)), rectally, transdermally, bucally, or nasally. The pharmaceutical compositions may also be prepared to be administered directly to the nervous system by routes including intracerebral, intraventricular, intracerebroventricular, intrathecal, intracisternal, intraspinal and/or peri-spinal route by delivery via intracranial or intravertebral needles and/or catheters with or without pump devices. Suitable compositions for oral administration include powders, granulates, aggregates, tablets, compressed or coated pills, dragees, sachets, hard or gelatin capsules, syrups and suspensions. Suitable compositions for parenteral administration include aqueous or non-aqueous solutions or emulsions, while for rectal administration suitable compositions for administration include suppositories with a hydrophilic or hydrophobic vehicle. For topical administration suitable transdermal delivery systems can be used and for nasal delivery suitable aerosol delivery systems can be used.

For example, in preparing the compositions for oral administration, any of the usual pharmaceutical media may be employed such as, for example, water, glycols, oils, alcohols in the case of oral liquid compositions such as suspensions, syrups, elixirs, emulsions and solutions; or solid carriers such as starches, sugars, kaolin, lubricants, binders, disintegrating agents in the case of solid compositions. For parenteral compositions, the carrier will usually comprise sterile water, at least in large part, though other ingredients, such as solubilizers, emulsifiers or further auxiliaries may be added thereto. Injectable solutions may be prepared in which the carrier comprises saline solution, glucose solution or a mixture of both. Injectable suspensions may also be prepared in which case appropriate liquid carriers, suspending agents may be employed. Also included are solid form preparations intended to be converted, shortly before use, to liquid form preparations such as powders for reconstitution. In the compositions suitable for percutaneous administration, the carrier optionally comprises a skin penetration enhancing agent and/or a wetting agent, optionally combined with suitable skin-compatible additives in minor proportions. The compound 6-b, or SV2A ligand, or combinations thereof, may also be administered via oral inhalation or insufflation by formulations suited for this type of administration such as a solution, a suspension or a dry powder. Suitable pharmaceutical compositions for administration in the form of aerosols or sprays are, for example, suspensions of the compound 6-b, or SV2A ligand, or both, in a pharmaceutically acceptable liquid carrier, such as ethanol or water, or a mixture thereof. If required, the formulation can also additionally contain other pharmaceutical auxiliaries such as surfactants, emulsifiers and stabilizers as well as a propellant. Such a preparation customarily contains the active compound in a concentration from approximately 0.1 to 50%, in particular from approximately 0.3 to 3% by weight.

The pharmaceutical compositions may contain the active ingredient compound 6-b, or the SV2A ligand, or both combined in a concentration of about 0.1% to about 50%, or about 1% to about 30%, or about 3% to about 20%, or about 5% to about 20%, all percentages being by weight, wherein the total of all components in said pharmaceutical compositions does not exceed 100%. In the compositions containing both two compounds, compound 6-b, and SV2A ligand, the compound 6-b, is present in a concentration of about 0.1% to about 50%, or about 1% to about 30%, or about 3% to about 20%, or about 5% to about 20%; and the SV2A ligand is present in a concentration of about 3% to about 50%, or about 5% to about 50%, or about 10% to about 50%, or about 10% to about 40%, or about 10% to about 30%, wherein the total of all components in said pharmaceutical compositions does not exceed 100%.

The pharmaceutical compositions may be conveniently presented in unit dosage form for ease of administration and uniformity of dosage. Examples include tablets (including scored or coated tablets), capsules, pills, suppositories, powder packets, wafers, injectable solutions or suspensions, and segregated multiples thereof. Of interest are solid dosage forms for oral administration such as tablets or capsules.

The solid dosage forms in unit dose form may be packed in any known package, blister packs being preferred, in particular for tablets and capsules. Where the compound 6-b, and SV2A ligand are formulated separately, they could be packed in separate blisters, but one blister could as well comprise unit dose forms of the compound 6-b, and of the SV2A ligand, for example one row with units of compound 6-b, and another with SV2A ligand. Other possibilities may be possible as well.

The combinations of this invention may be used to treat or prevent epilepsy and related disorders; neuropathic pain; migraine or resistant headache; and bipolar and related disorders; or they may be used as a neuroprotectant or to prevent epileptogenesis.

As used herein, the term **"treatment"** is intended to refer to all processes, wherein there may be a slowing, interrupting, arresting or stopping of the progression of a disease or an alleviation of symptoms, but does not necessarily indicate a total elimination of all symptoms

As used herein, unless otherwise noted, the terms **"epilepsy and related disorders"** or **"epilepsy or related disorder"** shall mean any disorder in which a subject (preferably a human adult, child or infant) experiences one or more seizures and / or tremors. Suitable examples include epilepsy (including localization-related epilepsies, generalized epilepsies, epilepsies with both generalized and local seizures), partial-onset seizures with or without generalization, myoclonic seizures, primary generalized tonic-clonic seizures in particular in patients with idiopathic generalized epilepsy, seizures associated with Lennox-Gastaut syndrome, seizures as a complication of a disease or condition (such as seizures associated with encephalopathy, phenylketonuria, juvenile Gaucher's disease, Lundborg's progressive myoclonic epilepsy, stroke, head trauma, stress, hormonal changes, drug use or withdrawal, alcohol use or withdrawal, sleep deprivation, fever, infection), status epilepticus (convulsive or non convulsive), essential tremor, restless limb syndrome. Preferably, the disorder is selected from epilepsy (regardless of type, underlying cause or origin), essential tremor or restless limb syndrome. More preferably, the disorder is epilepsy (regardless of type, underlying cause or origin) or essential tremor. A particular example of epilepsy is refractory epilepsy, also referred to as treatment or therapy resistant epilepsy. This term is often used when patients have failed three or more anti-epileptic drugs (AEDs). Refractory epilepsy also includes refractory partial epilepsy and refractory generalized epilepsy (including idiopathic or symptomatic).

As used herein, the term **"neuropathic pain"** includes pain resulting from chronic or debilitating conditions or disorders. The chronic or debilitating conditions or disorders which can lead to neuropathic pain include painful diabetic peripheral neuropathy, post-herpetic neuralgia, trigeminal neuralgia, post-stroke pain, multiple sclerosis-associated pain, neuropathies-associated pain such as in idiopathic or post-traumatic neuropathy and mononeuritis, HIV-associated neuropathic pain, cancer-associated neuropathic pain, carpal tunnel-associated neuropathic pain, spinal cord injury-associated pain, complex regional pain syndrome, fibromyalgia-associated neuropathic pain, lumbar and cervical pain, reflex sympathic dystrophy, phantom limb syndrome and other chronic and debilitating condition-associated pain syndromes.

As used herein, the term **"migraine"** shall mean a chronic, episodic and debilitating clinical condition that is diagnosed by the presence of moderate to severe pulsating unilateral headaches lasting between 4 and 72 h, which includes migraine without aura and migraine with aura. As used herein, "migraine without aura" shall mean at least five attacks fulfilling the following criteria: (a) the headache attack lasts 4-72 hours with the headache having at least two of the following features: unilateral location, pulsating quality, moderate or severe intensity with direct influence on activities of daily living, and aggravation by walking up stairs or similar routines: and (b) during the headache at least one of the following occurs: nausea and/or vomiting, and photophobia and phonophobia. As used herein, "migraine with aura" shall mean at least two attacks accompanied by at least 3 of the 4 following features: (a) one or more fully reversible aura symptoms: (b) at least one aura symptom which develops gradually over more than four minutes or two or more symptoms which occur in succession; (c) no aura symptom which lasts more than 60 minutes; (d) a headache occurs prior to, simultaneously with or following the aura, with a free interval between aura and headache of less than about 60 minutes.

As used herein, the term **"bipolar and related disorders"** shall include bipolar disorder I (e.g. single manic episode, most recent episode hypomanic, most recent episode manic, most recent episode mixed, most recent episode depressed and most recent episode unspecified), bipolar disorder II, cyclothymic disorder and bipolar disorder not otherwise specified (as these terms are defined by their diagnostic criteria, in the Diagnostic and Statistical manual of Mental Disorders 4th Edition, Text Revision, American Psychiatric Association, 2000 (DSM-IV-TR) or in the 5th Edition, Text Revision, American Psychiatric Association, 2013 (DSM-5™). Preferably, the bipolar disorder is characterized by depressive and manic (or hypomanic) phases, wherein the phases cycle. Preferably, the bipolar disorder is bipolar disorder I or bipolar disorder II. As used herein "mania" shall include mania or a manic mood phase, regardless of underlying cause. As used herein, the term "bipolar mania" is intended to mean the mania associated with, characteristic of or symptomatic of a bipolar disorder. Thus, methods of treating bipolar mania of the present invention are directed to methods which treat the mania and/or manic phase of bipolar disorders. As used herein, the term "bipolar depression" is intended to mean the depression associated with, characteristic of or symptomatic of a bipolar disorder. Thus, methods of treating bipolar depression of the present invention are directed to methods which treat the depression and/or depressed phase of bipolar disorders. As used herein, unless otherwise noted the terms "cycling" or "bipolar cycling" shall refer to the alteration of mood between depressive and manic phases characteristic of bipolar disorders. Thus, the present invention includes methods for the stabilization of said cycling, including decreasing the frequency of the cycling and/or decreasing the magnitude of the manic and/or depressive phases.

Thus, in an embodiment, the pharmaceutical composition of the present invention may be used for mood stabilization, in particular mood stabilization for manic depression. As used herein, the term **"epileptogenesis"** refers to the gradual process by which epilepsy develops. This process may occur following brain insults or a variety of conditions, including neurodegenerative diseases, traumatic brain injury, stroke, brain tumor, infections of the central nervous system, and status epilepticus; or it may occur following gene mutations.

As used herein, the term **"anxiety"** refers in particular to generalized anxiety disorder. As used herein, the term **"about"** has its conventional meaning. In particular embodiments, when in relation to a numerical value, it may be interpreted to mean the numerical value ± 10%, or ± 5%, or ± 2%, or ± 1%, or ± 0.5 %, or ± 0.1%. In other embodiments, the precise value is meant, i.e. by leaving out the word "about". **"And/or"** means that each one or both or all of the components or features of a list are possible variants, especially two or more thereof in an alternative or cumulative way. As used herein, the term **"a", "an", "the"** and similar terms used in the context of the present invention (especially in the context of the claims) are to be construed to cover both the singular and plural unless otherwise indicated herein or clearly contradicted by the context.

### EXAMPLES

The following Examples are set forth to aid in the understanding of the invention.

### A) COMPOUNDS - CHEMISTRY AND IN VITRO TESTING

Unless otherwise noted, all starting materials were obtained from commercial suppliers and used without further purification.

Hereinafter, "aq." means aqueous; "DCE" means 1,2-dichloroethane, "DCM" means dichloromethane; "DIPE" means diisopropylether; "DIPEA" means *N,N-*diisopropylethylamine; "DMF" means *N*,*N*-dimethylformamide; "ES" means electrospray; "Et₃N" means triethylamine; "Et₂O" means diethyl ether; "EtOAc" means ethyl acetate; "h" means hours; "HPLC" means high performance liquid chromatography; "HRMS" means high-resolution mass spectra/spectrometry; "1" or "L" means liter; "LRMS" means low-resolution mass spectrometry/spectra; "MeOH" means methanol; "min" means minute(s); "mp" means melting point; "Pd(PPh₃)₄" means tetrakis(triphenylphosphine)palladium(0); "RP" means reverse phase; "r.t." means room temperature; "s" means seconds; "sat." means saturated; "SFC" means supercritical fluid chromatography; "sol." means solution; "THF" means tetrahydrofuran.

Microwave assisted reactions were performed in a single-mode reactor: Initiator™ Sixty EXP microwave reactor (Biotage AB), or in a multimode reactor: MicroSYNTH Labstation (Milestone, Inc.).

Thin layer chromatography (TLC) was carried out on silica gel 60 F254 plates (Merck) using reagent grade solvents. Open column chromatography was performed on silica gel, particle size 60 Å, mesh = 230-400 (Merck) using standard techniques. Automated flash column chromatography was performed using ready-to-connect cartridges from Merck, on irregular silica gel, particle size 15-40 µm (normal phase disposable flash columns) on a SPOT or LAFLASH system from Armen Instrument.

The absolute stereochemical configuration for some of the compounds was determined using vibrational circular dichroism (VCD). They were measured on a Bruker Equinox 55 equipped with a PMA 37, in a KBr liquid cell using CD₂Cl₂ as solvent (PEM: 1350cm-1, LIA: 1mV, resolution: 4 cm⁻¹). A description on the use of VCD for the determination of absolute configuration can be found in Dyatkin A.B. et. al, Chirality, 14:215-219 (2002).

Whenever the notation "RS" is indicated herein, it denotes that the compound is a racemic mixture, unless otherwise indicated. The stereochemical configuration for some compounds has been designated "*R*" or "*S*" when the mixture was separated The enantiomeric excess of compounds reported herein was determined by analysis of the racemic mixture by supercritical fluid chromatography (SFC) followed by SFC comparison of the separated enantiomer(s).

### Preparation of intermediates

### Description 1 - Intermediate 1

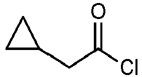

Cyclopropylacetic acid ([CAS 5239-82-7], 50 g, 500 mmol) was dissolved in CH₂Cl₂ (300 mL) then SOCl₂ (100 mL) was added. The reaction mixture was stirred at 60 °C for 2 h and then the solvent was evaporated to yield intermediate **1** (53 g, 90%), which was used without further purification.

### Description 2 - Intermediate 2

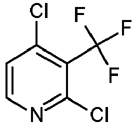

To a solution of 2,4-dichloro-3-iodopyridine ([CAS 343781-36-2], 290 g, 1058 mmol) in DMF (1.7 L) was added methyl 2,2-difluoro-2-(fluorosulfonyl)acetate ([CAS 680-15-9], 403 g, 2098 mmol) and CuI (403 g, 2.13 mol), the reaction was then heated at 100 °C for 5 h.

The reaction was cooled and filtered. The filtrate was diluted with H₂O and extracted with Et₂O and washed with a NH₃ solution. The organic layer was dried (Na₂SO₄), filtered and concentrated *in vacuo* to yield intermediate **2** (160 g), which was used without further purification.

### Description 3 - Intermediate 3

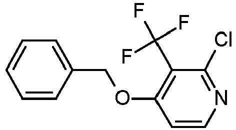

To a solution of NaH (60% in oil, 24 g, 600 mmol) in DMF (2 L) at 0 °C was added benzyl alcohol (35 g, 325 mmol), then the reaction was stirred for 2 min. Intermediate **2** (160 mg, 741 mmol) was added in one portion, and stirred at 0 °C for 1 h. The reaction was diluted by the addition of H₂O and extracted with Et₂O. The organic layer was dried (Na₂SO₄), filtered and concentrated *in vacuo.* The residue was purified by column chromatography over silica gel (eluent: petroleum ether/EtOAc = 20/1). The pure fractions were collected and the solvent was evaporated to yield intermediate **3** (100 g, 38%).

### Description 4 - Intermediate 4

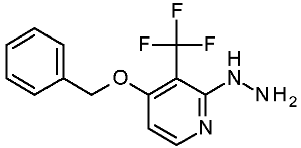

To a solution of intermediate **3** (100 g, 277 mmol) in 1,4-dioxane (1.5 L) was added NH₂NH₂ hydrate (85% solution in water, 300 g, 9.11 mol), the reaction was then heated in sealed tube at 160 °C for 2 h. The mixture was concentrated *in vacuo,* dissolved in DCM, washed with NaHCO₃. The organic layer was dried (Na₂SO₄), filtered and concentrated *in vacuo* to yield intermediate **4** (90 g, 90%), which was used without further purification.

### Description 5 - Intermediate 5

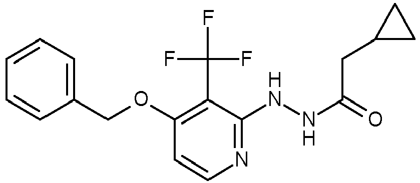

To a solution of intermediate **4** (90 g, 318 mmol) in CH₂Cl₂ (1.5 L) was added triethylamine (64.3 g, 636 mmol), the mixture was cooled to 0 °C, then a solution of intermediate **1** (53 g, 449 mmol) in CH₂Cl₂ was added. The solution was stirred at RT for 1 h. The reaction mixture was washed with a sat. aq. sol. of NaHCO₃, and extracted with CH₂Cl₂. The organic layer was dried (Na₂SO₄), filtered and concentrated *in vacuo* to yield intermediate **5** (104.4 g, 90%).

### Description 6

### Intermediate 9

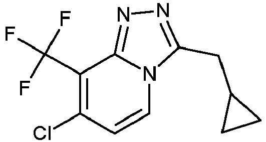

To a solution of intermediate **5** (101 g, 277 mmol) in CH₃CN (1.2 L) were added phosphorus(V) oxychloride (84.7 g, 553 mmol) and *N*,*N*-diisopropylethylamine (71.3 g, 553 mmol). The reaction mixture was stirred at 90 °C for 38 h. The reaction was then diluted with DCM and washed with a Na₂CO₃ solution. The organic layer was dried (Na₂SO₄), filtered and concentrated *in vacuo.* The residue was purified by column chromatography over silica gel (eluent: petroleum ether/EtOAc = 4/1). The pure fractions were collected and the solvent was evaporated to yield intermediate **9** (31.39 g, 41%).

### Description 7 - Intermediate 13

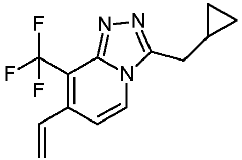

(Ph₃P)₄Pd (2.096 g, 1.81 mmol) was added to a stirred solution of intermediate **9** (10 g, 36.28 mmol) and 4,4,5,5-tetramethyl-2-vinyl-1,3,2-dioxoborolane ([CAS 75927-49-0], 7.77 mL, 43.53 mmol) in deoxygenated dioxane (30 mL) and a deoxygenated NaHCO₃ saturated solution (30 mL) under nitrogen. The mixture was stirred at 100 °C for 18 h. The mixture was diluted with EtOAc/water and filtered through a pad of diatomaceous earth. The filtrate was treated with brine and extracted with EtOAc. The organic layer was separated, dried (Na₂SO₄), filtered and the solvents evaporated *in vacuo.* The crude product was purified by flash column chromatography (silica; EtOAc in CH₂Cl₂ 0/100 to 5/95). The desired fractions were collected and concentrated *in vacuo* to yield intermediate **13** (6.08, 63%) as a yellow solid.

### Description 8

### Intermediate 17

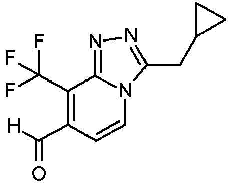

Osmium tetraoxide (2.5% in t-BuOH, 10.103 mL, 0.781 mmol) and then, sodium periodate 12.53 g, 58.58 mmol) in water (48.5 mL) were added to a suspension of Intermediate **13** (6.08 g, 20.02 mmol) in dioxane (192 mL). The mixture was stirred at room temperature for 2 h.

The mixture was treated with water and EtOAc and it was filtered off through a pad of diatomaceous earth. The filtrate was extracted with EtOAc. The organic layer was separated, dried (Na₂SO₄), filtered and the solvents evaporated *in vacuo.* The crude product was washed with Et₂O and it was filtered and dried to yield intermediate **17** (4.25 g, 79%) as a brown solid.

### Description 9

### (a) Intermediates 21a, 21b and 21c

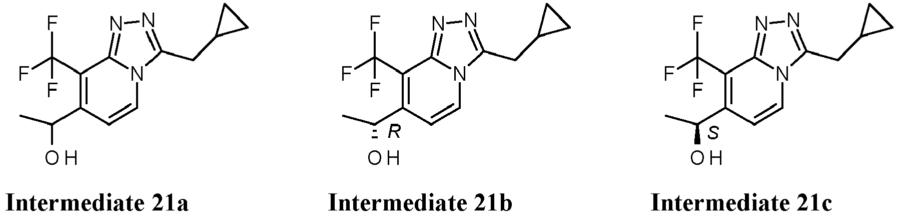

Methylmagnesium bromide (1.4 M in THF, 12.40 mL, 17.37 mmol) was added dropwise to a stirred suspension of intermediate **17** (4.25 g, 15.79 mmol) in THF (281.07 mL) at -20 °C under N₂ atmosphere. The mixture was stirred at -20 °C for 45 minutes. The crude was treated with a sat. sol. of NH₄Cl and extracted with EtOAc. The organic layer was separated, dried (Na₂SO₄), filtered and concentrated *in vacuo.* The residue was purified by flash column chromatography (silica; MeOH in DCM 0/100 to 4/96). The desired fractions were collected and concentrated *in vacuo* to yield intermediate **21a** (racemic mixture) (2.96 g, 66%). Intermediate **21a** (1.82 g) was purified by chiral SFC: [Stationary phase: CHIRALPAK AD-H (5µm 250 x20 mm), Mobile phase: 80% CO₂, 20% EtOH] yielding **21b** (*R*-enantiomer) (0.453 g, 10%) as a pale grey solid and intermediate **21c** (*S*-enantiomer) (0.439 g, 10%).

### Preparation of the final compound

### Example 1

### (a) Synthesis of compounds 4-b, 6-b and 5-b

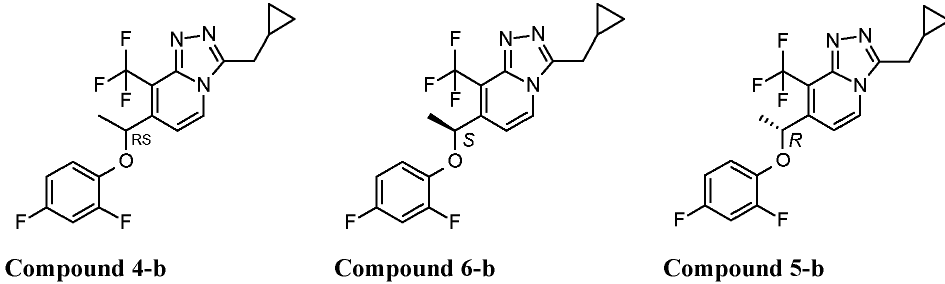

DIAD (2.07 mL, 10.52 mmol) was added dropwise to a stirred solution of intermediate **21a** (2 g, 7.01 mmol), 2,4-difluorophenol (1.00 mL, 10.52 mmol) and triphenylphosphine (2.76 g, 10.52 mmol) in THF (74.18 mL) at 0 °C and under nitrogen atmosphere. The mixture was stirred at 100 °C for 10 minutes under microwave irradiation. The mixture was diluted with EtOAc and washed with a sat. sol. of NaHCO₃. The organic layer was separated, dried (Na₂SO₄), filtered and concentrated *in vacuo.* The residue was purified by flash column chromatography (silica; MeOH in DCM 0/100 to 97/3). The desired fractions were collected and concentrated *in vacuo.* The residue was triturated with DIPE to give compound **4-b** (1.46 g, 52%) as a white solid, which was purified by chiral SFC [Stationary phase: Chiralpak AD (5µm 250^{∗}30 mm, Mobile phase: 85% CO₂, 15% iPrOH)], yielding compound **6-b** (0.659 g, 24%) and compound **5-b** (0.693 g, 25%).

### (b) Alternative synthesis of compound 6-b

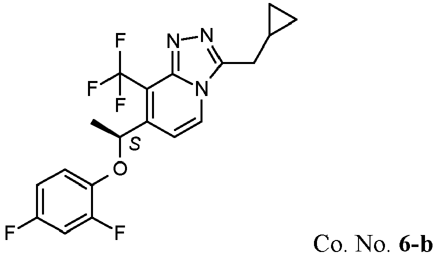

DIAD (31.06 µL, 0.16 mmol) was added dropwise to a stirred solution of intermediate **21b** (30 mg, 0.11 mmol), 2,4-difluorophenol (15.07 µL, 0.16 mmol) and triphenylphosphine (41.38 mg, 0.16 mmol) in THF (1.11 mL) at 0 °C and under nitrogen atmosphere. The mixture was stirred at 100 °C for 10 minutes under microwave irradiation. The mixture was diluted with EtOAc and washed with a sat. sol. of NaHCO₃. The organic layer was separated, dried (Na₂SO₄), filtered and concentrated *in vacuo.* The residue was purified by flash column chromatography (silica; MeOH in DCM 0/100 to 97/3). The desired fractions were collected and concentrated *in vacuo.* The residue was triturated with DIPE to give compound **6-b** (40 mg, 96%) as a white solid.

### (c) Synthesis of compound 6-b hydrochloride salt (.HCl)

DIAD (207.06 µL, 1.05 mmol) was added dropwise to a stirred solution of intermediate **21b** (200 mg, 0.70 mmol), 2,4-difluorophenol (100.45 µL, 1.05 mmol) and triphenylphosphine (275.84 mg, 1.0516 mmol) in THF (4 mL) at 0 °C and under nitrogen atmosphere. The mixture was stirred at 100 °C for 15 minutes under microwave irradiation. The mixture was diluted with EtOAc and washed with a sat. sol. of NaHCO₃. The organic layer was separated, dried (Na₂SO₄), filtered and concentrated in vacuo. The residue was purified by RP HPLC (Stationary phase: C18 XBridge 30 x 100 mm 5 µm, Mobile phase: Gradient from 60% 0.1% NH₄CO₃H/NH₄OH pH 9 solution in Water, 40% CH₃CN to 43% 0.1% NH₄CO₃H/NH₄OH pH 9 solution in Water, 57% CH₃CN), yielding a white solid residue that was dissolved in Et₂O (8 mL) and 1,4-dioxane (0.5 mL). To the solution thus obtained HCl (4M in dioxane, 200 µL) was added dropwise. The white solid precipitate was filtered, washed with Et₂O, dried (Na₂SO₄) and evaporated under vacuum. The white residue thus obtained was triturated with Et₂O to give compound **6-b** .HCl (110 mg, 36%) as a white solid.

### Analytical part

### Optical Rotations

Optical rotations were measured on a Perkin-Elmer 341 polarimeter with a sodium lamp and reported as follows: [α]° (λ, c g/100ml, solvent, T°C).

[α]_{λ}^{T} = (100α) / (*l* x *c*) : where *l* is the path length in dm and c is the concentration in g/100 ml for a sample at a temperature T (°C) and a wavelength λ (in nm). If the wavelength of light used is 589 nm (the sodium D line), then the symbol D might be used instead. The sign of the rotation (+ or -) should always be given. When using this equation the concentration and solvent are always provided in parentheses after the rotation. The rotation is reported using degrees and no units of concentration are given (it is assumed to be g/100 ml).

### LCMS

For (LC)MS-characterization, the following methods were used.

### General procedure

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Compounds are described by their experimental retention times (Rₜ) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl..), the reported value is the one obtained for the lowest isotope mass. All results were obtained with experimental uncertainties that are commonly associated with the method used. Hereinafter, "SQD" means Single Quadrupole Detector, "RT" room temperature, "BEH" bridged ethylsiloxane/silica hybrid, "HSS" High Strength Silica, "DAD" Diode Array Detector.

**Table B: LCMS Method codes (Flow expressed in mL/min; column temperature (T) in °C; Run time in minutes).**

| **Instrument** | **Column** | **Mobile phase** | **Gradient** | $\begin{matrix}Flow \\ Col T\end{matrix}$ | **Run time** | **LCMS Method** |
|---|---|---|---|---|---|---|
| Waters: Acquity® UPLC® - DAD and SQD | Agilent: Eclipse Plus C18 RRHD (1.8µm, 2.1x50mm) | A: 95% CH₃COONH₄ 6.5mM + 5% CH₃CN, B: CH₃CN | From 95% A to 5% A in 4.6min, held for 0.4min | $\begin{matrix}1 \\ 50\end{matrix}$ | 5 | 1 |
| Waters: Acquity UPLC® - DAD and Quattro Micro™ | Waters: BEH C18 (1.7µm, 2.1x100mm) | A: 95% CH₃COONH₄ 7mM / 5% CH₃CN, B: CH₃CN | 84.2% A for 0.49min, to 10.5% A in 2.18min, held for 1.94min, back to 84.2% A in 0.73min, held for 0.73min. | $\begin{matrix}0.343 \\ 40\end{matrix}$ | 6.2 | 3 |

### Melting points

Values are peak values, and are obtained with experimental uncertainties that are commonly associated with this analytical method.

### Mettler FP 81HT/FP90 apparatus

For a number of compounds, melting points were determined in open capillary tubes on a FP 81HT / FP90 apparatus (Mettler-Toledo). Melting points were measured with a temperature gradient of 1, 3, 5 or 10 °C/minute. Maximum temperature was 300 °C. The melting point was read from a digital display.

**Table C: Physico-chemical data, retention time (Rₜ) in min, [M+H]⁺ peak (protonated molecule), LCMS method and mp (melting point in °C). (n.d. = not determined).**

| **Co. no.** | **Mp (°C)** | **Rₜ (min)** | **[MH⁺]** | **LCMS method** | **Optical Rotation** |
|---|---|---|---|---|---|
| **4-b** | 121.7 | 2.41 | 398 | 1 | |
| **5-b** | 142 | 2.99 | 398.3 | 3 | +95.7 ° (589 nm, c 0.69 w/v%, DMF, 20 °C) |
| **6-b** | 142.4 | 2.99 | 398.2 | 3 | -95.4 ° (589 nm, c 0.7 w/v%, DMF, 20 °C) |

### SFC-MS

### General procedure

The SFC measurement was performed using Analytical system from Berger instrument comprising a FCM-1200 dual pump fluid control module for delivering carbon dioxide (CO2) and modifier, a CTC Analytics automatic liquid sampler, a TCM-20000 thermal control module for column heating from room temperature to 80°C. An Agilent 1100 UV photodiode array detector equipped with a high-pressure flow cell standing up to 400 bars was used. Flow from the column was split to a MS spectrometer. The MS detector was configured with an atmospheric pressure ionization source.The following ionization parameters for the Waters ZQ mass spectrophotometer are: corona: 9µa, source temp: 140°C, cone: 30 V, probe temp 450°C, extractor 3 V, desolvatation gas 400L/hr, cone gas 70 L/hr. Nitrogen was used as the nebulizer gas. Data acquisition was performed with a Waters-Micromass MassLynx-Openlynx data system.

*Method 1*: In addition to the general procedure: The analytical chiral separation in SFC-MS was carried out on a CHIRALPAK AD DAICEL column (10 µm, 4.6 x 250 mm) at 35 °C with a flow rate of 3.0 ml/min. The mobile phase is 85% CO₂, 15% iPrOH (+ 0.3% iPrNH₂) hold 7 min in isocratic mode.

**Table D: Analytical SFC data - Rₜ means retention time (in minutes), [M+H]⁺ means the protonated mass of the compound, method refers to the method used for SFC/MS analysis of enantiomerically pure compounds. The measurement was compared against the mixture.**

| **Co. Nr.** | **Rₜ** | **[M+H]⁺** | **UV Area %** | **Method** | **Isomer Elution Order*** |
|---|---|---|---|---|---|
| **6-b** | 4.28 | 398 | 100 | 1 | A |
| **5-b** | 5.98 | 398 | 100 | 1 | B |

| | | | | | |
|---|---|---|---|---|---|
| *A means the first isomer that elutes. B means the second isomer that elutes. | | | | | |

### Nuclear Magnetic Resonance (NMR)

¹H NMR spectra were recorded on a Bruker DPX-400 spectrometer with standard pulse sequences, operating at 400 MHz and 500 MHz respectively. Chemical shifts (δ) are reported in parts per million (ppm) downfield from tetramethylsilane (TMS), which was used as internal standard.
**Co. No. 6-b:** ¹H NMR (400 MHz, CDCl₃) δ ppm 0.30 - 0.38 (m, 2 H), 0.59 - 0.68 (m, 2 H), 1.14 - 1.22 (m, 1 H), 1.72 (d, *J*=6.5 Hz, 3 H), 3.02 - 3.14 (m, 2 H), 5.84 (q, *J*=6.3 Hz, 1 H), 6.67 - 6.73 (m, 1 H), 6.80 - 6.89 (m, 2 H), 7.30 (d, *J*=7.4 Hz, 1 H), 8.11 (d, *J*=7.4 Hz, 1 H)

### In vitro testing

The compounds provided in the present invention are positive allosteric modulators of mGluR2. These compounds appear to potentiate glutamate responses by binding to an allosteric site other than the glutamate binding site. The response of mGluR2 to a concentration of glutamate is increased when compounds are present. Compound 6-b is expected to have its effect substantially at mGluR2 by virtue of its ability to enhance the function of the receptor. The effects of positive allosteric modulators tested at mGluR2 using the [³⁵S]GTPγS binding assay method described below and which is suitable for the identification of such compounds, and more particularly compound 6-b, are shown in Table E.

### [³⁵SIGTPγS binding assay

The [³⁵S]GTPγS binding assay is a functional membrane-based assay used to study G-protein coupled receptor (GPCR) function whereby incorporation of a non-hydrolysable form of GTP, [³⁵S]GTPγS (guanosine 5'-triphosphate, labelled with gamma-emitting ³⁵S), is measured. The G-protein α subunit catalyzes the exchange of guanosine 5'-diphosphate (GDP) by guanosine triphosphate (GTP) and on activation of the GPCR by an agonist, [³⁵S]GTPγS, becomes incorporated and cannot be cleaved to continue the exchange cycle (Harper (1998) Current Protocols in Pharmacology 2.6.1-10, John Wiley & Sons, Inc.). The amount of radioactive [³⁵S]GTPγS incorporation is a direct measure of the activity of the G-protein and hence the activity of the agonist can be determined. mGlu2 receptors are shown to be preferentially coupled to Gai-protein, a preferential coupling for this method, and hence it is widely used to study receptor activation of mGlu2 receptors both in recombinant cell lines and in tissues. Here we describe the use of the [³⁵S]GTPγS binding assay using membranes from cells transfected with the human mGlu2 receptor and adapted from Schaffhauser et al. (Molecular Pharmacology, 2003, 4:798-810) for the detection of the positive allosteric modulation (PAM) properties of the compounds of this invention.

### Membrane preparation

CHO-cells were cultured to pre-confluence and stimulated with 5 mM butyrate for 24 h. Cells were then collected by scraping in PBS and cell suspension was centrifuged (10 min at 4000 RPM in benchtop centrifuge). Supernatant was discarded and pellet gently resuspended in 50 mM Tris-HCl, pH 7.4 by mixing with a vortex and pipetting up and down. The suspension was centrifuged at 16,000 RPM (Sorvall RC-5C plus rotor SS-34) for 10 minutes and the supernatant discarded. The pellet was homogenized in 5 mM Tris-HCl, pH 7.4 using an ultra-turrax homogenizer and centrifuged again (18,000 RPM, 20 min, 4 °C). The final pellet was resuspended in 50 mM Tris-HCl, pH 7.4 and stored at -80 °C in appropriate aliquots before use. Protein concentration was determined by the Bradford method (Bio-Rad, USA) with bovine serum albumin as standard.

### [³⁵S]GTPγS binding assay

Measurement of mGluR2 positive allosteric modulatory activity of test compounds was performed as follows. Test compounds and glutamate were diluted in assay buffer containing 10 mM HEPES acid, 10 mM HEPES salt, pH 7.4, 100 mM NaCl, 3 mM MgCl₂ and 10 µM GDP. Human mGlu2 receptor-containing membranes were thawed on ice and diluted in assay buffer supplemented with 14 µg/ml saponin. Membranes were pre-incubated with compound alone or together with a predefined (∼EC₂₀) concentration of glutamate (PAM assay) for 30 min at 30 °C. After addition of [³⁵S]GTPγS (f.c. 0.1 nM), assay mixtures were shaken briefly and further incubated to allow [³⁵S]GTPγS incorporation on activation (30 minutes, 30 °C). Final assay mixtures contained 7 µg of membrane protein in 10 mM HEPES acid, 10 mM HEPES salt, pH 7.4, 100 mM NaCl, 3 mM MgCl₂, 10 µM GDP and 2 µg/ml saponin. Total reaction volume was 200 µl. Reactions were terminated by rapid filtration through Unifilter-96 GF/B plates (Perkin Elmer, Massachusetts, USA) using a 96-well filtermate universal harvester. Filters were washed 6 times with ice-cold 10 mM NaH₂PO₄/10 mM Na₂HPO₄, pH 7.4. Filters were then air-dried, and 40 µl of liquid scintillation cocktail (Microscint-O) was added to each well. Membrane-bound radioactivity was counted in a Microplate Scintillation and Luminescence Counter from Perkin Elmer.

### Data analysis

The concentration-response curves of representative compounds of the present invention -obtained in the presence of EC₂₀ of mGluR2 agonist glutamate to determine positive allosteric modulation (PAM)- were generated using the Lexis software interface (developed at J&J). Data were calculated as % of the control glutamate response, defined as the maximal response that is generated upon addition of glutamate alone. Sigmoid concentration-response curves plotting these percentages versus the log concentration of the test compound were analyzed using non-linear regression analysis. The concentration producing half-maximal effect is then calculated as EC₅₀.
The pEC₅₀ values below were calculated as the -log EC₅₀, when the EC₅₀ is expressed in M. Eₘₐₓ is defined as relative maximal effect (i.e. maximal % effect relative to the control glutamate response).
Table E below shows the pharmacological data obtained for compound 6-b and current pharmacological data obtained for reference compounds.

**Table E: Pharmacological data for compounds according to the invention.**

| **Co. No.** | **GTPγS** - **hmGluR2 PAM pEC₅₀** | **GTPγS** - **hmGluR2 PAM Emax** |
|---|---|---|
| **6-b** | 7.39 | 256 |
| **5-b** | 6.06 | 141 |
| **4-b** | 7.04 | 329 |
| **Ref. 1** | 7.11 | 258 |
| **Ref. 1a** | 6.95 | 286 |
| **Ref. 2** | 7.82 | 290 |
| **Ref. 2a** | 7.61 | 484 |
| **Ref. 2-a** | 6.84 | 340 |
| **Ref. 25-a** | 7.07 | 261 |

All compounds were tested in presence of mGluR2 agonist glutamate at a predetermined EC₂₀ concentration, to determine positive allosteric modulation . pEC₅₀ values were calculated from a concentration-response experiment of at least 8 concentrations.

### B) ANTICONVULSANT STUDIES AND REFERENC STUDIES WITH mGluR2 COMPOUNDS (ORTHOSTERIC AGONIST AND PAMs)

### General

### Preparation of test compounds and solutions

Test compounds were administered using an optimal fluid volume to body fluid ratio. Test compounds were administered to mice in a volume of 0.01 mL/g of body weight (White H.S., et al., General Principles: Experimental selection, quantification, and evaluation of antiepileptic drugs, in Antiepileptic Drugs, Fourth Edition, R. H. Levy, R.H. Mattson, and B. S. Meldrum, Editors. 1995, Raven Press, Ltd.: New York, pp. 99-110). For subcutaneous (s.c.) administration, the test compounds were administered into a loose fold of skin along the back of the animal except compound **6-b,** which was administered orally (p.o). For each of the tests performed on the test compounds (except on compound **6-b**), final compound concentrations were administered as aqueous solution in 20% Hp-β-CD. For compound **6-b,** a 40% Hp-β-CD stock solution was first prepared and utilized for formulating compound **6-b** at the desired concentrations for testing via the oral route; final compound concentrations were administered as suspensions in 20% Hp-β-CD. A 20% Hp-β-CD solution was used for the vehicle groups.

For reference compound LY-404039, final compound concentrations were administered as a saline solution s.c..

For reference compound CAS 1092453-15-0, final compound concentrations were administered in 10% Hp-β-CD (+NaCl) vehicle following dissolution.

Final levetiracetam concentrations were administered in a 0.5% methylcellulose (MC) aqueous solution administered by intraperitoneal (i.p.) injection.

### Critical Reagents

### a) Vehicle Solutions

### 0.5% Methylcellulose (MC)

### 40% Hydroxypropyl-β-cyclodextrin (Hp-β-CD) stock solution

### b) Miscellaneous Solutions

Tetracaine (0.5% solution w/v) was added dropwise from a plastic dropper bottle onto the eyes of all animals that would subsequently receive electrical stimulation via corneal electrodes.

### Animals and Animal Husbandry

Adult male CF No 1 albino mice (26-35 g) were obtained from Charles River, Portage, Michigan. The animals were maintained on an adequate diet (Prolab RMH 3000) and allowed free access to food and water, except during the short time they were removed from their cage for testing. Animals newly received in the laboratory were allowed sufficient time to correct for possible food and water restriction incurred during transit before being employed in testing. All mice were housed in plastic cages in specially constructed rooms with controlled humidity, exchange of air and controlled lighting (12 hours on - 12 hours off). The animals were housed, fed, and handled in a manner consistent with the recommendations in the National Council Publication, "Guide for the Care and Use of Laboratory Animals".

### Minimal Motor Impairment (MMI)

Acute MMI was assessed by a combination of direct observations of the animal for overt symptoms of the animal's neurological or muscular function. In mice, the rotarod procedure was used to disclose minimal muscular or neurological impairment. When a mouse is placed on a rod that rotates at a speed of 6 rpm, the animal can maintain its equilibrium for long periods of time. The animal was considered toxic if it fell off this rotating rod three times during a 1 min period.

### Determination of Median Effective and Toxic Doses (ED₅₀ and TD₅₀)

In the determination of an ED₅₀ or TD₅₀ for each test compound, the first dose administered is usually the same dose as that used in a successful TPE determination. If the initial dose employed was effective or toxic in more than 50% of animals, the next dose would be one-half that of the initial dose; if the initial dose was effective or toxic in less than 50% of animals, the following dose would be twice that of the initial dose. Third and fourth doses were selected to produce an evenly spaced dose response line. There should be a minimum of 4 points either including or lying between 0 and 100%.

### TPE Determination

Groups of generally four animals each were administered test compounds and each group was tested at one of five time points: 0.25, 0.5, 1, 2, or 4h post-treatment (White et al. 1995). TPE was determined using the 6Hz (32mA) assay. The time (0.25, 0.5, 1, 2, or 4h post-treatment) at which maximal protection was observed was considered the Time of Peak Effect (TPE).

At the TPE determined for this study, or determined previously, compounds were tested in the 6Hz assay (32 and/or 44 mA), across several doses and comprising doses that elicited little or no protection to full protection.

An ED₅₀ and 95% confidence interval (C.I.) were calculated using Probit analysis on a computer program provided in the laboratory (Finney "Probit Analysis" 34d Ed 1971, London: Cambridge University Press).

### Serum Collection for pK/pD Analysis

In various tests, animals were sacrificed following testing, and trunk blood and/or brain tissue (whole brains) was collected for quantification of drug levels. Immediately after testing, animals were decapitated and trunk blood was collected into a BD Vacutainer® tube containing K2EDTA and chilled on ice until centrifugation. Following centrifugation (13000 - 18000 rpm, 5-7 min), the plasma was removed and transferred to a labeled microcentrifuge tube and stored at -80 °C. For brain tissue collection, brains were removed immediately following decapitation and flash frozen. The frozen sample was placed in a labeled centrifuge tube and stored at -80 °C.

### 6 Hz Psychomotor Seizure Test in Mice

The 6Hz seizure test is used as a model of pharmacoresistant limbic seizures. The 6Hz seizure displays a resistance to phenytoin, carbamazepine, lamotrigine, and topiramate (Barton et al. "Pharmacological characterization of the 6 Hz psychomotor seizure model of partial epilepsy" Epilepsy Research 2001, Vol. 47, pp. 217-222).

### Method for 6 Hz Psychomotor Seizure Test

Focal seizures were induced in mice via corneal stimulation (6Hz, 0.2 msec rectangular pulse, 3 sec duration; Barton et al. 2001). Mice were tested at either 32mA or 44mA. Prior to stimulation, drops of 0.5% tetracaine were applied to each eye. The seizures that arise from corneal stimulation in this assay are characterized by a minimal clonic phase followed by stereotyped automatistic behaviors including stun, forelimb clonus, twitching of the vibrissae, and Straub-tail. Animals not displaying these behaviors were considered protected.

### EXAMPLE 1 - STUDIES WITH REFERENCE COMPOUNDS 1 AND 2

### 1.1. Combination Study with reference Co. No. 1, Co. No. 2 and Levetiracetam

First, each compound was tested individually at a dose that displayed minimal activity in the 6Hz 44 mA test at each compound's TPE. When the mGluR2 PAM compounds and levetiracetam were administered in combination (same dose and time-point as individual tests) nearly complete protection was observed in the 6Hz 44 mA test (Table 2). In addition to recording the efficacy and toxicity data for these compounds alone or in combination, both plasma and brain samples were collected from each of the groups for pharmacokinetic/pharmacokinetic analysis. No pharmacokinetic interaction was observed based on compound levels in the plasma and brain samples (data not shown). In summary, reference compounds 1 and 2 displayed positive pharmacodynamic interaction with levetiracetam in the 6Hz model that does not appear to be due to pharmacokinetic interaction, and without increasing motor impairment (Tables 2, 2a, 2b). The effect of 1 dose of reference Compound **2** was also tested on the dose-response of LEV. As shown in Table 3, there was a ∼200-fold shift in the ED₅₀ of LEV compared to when LEV was tested alone. LEV seemed to increase the potency of reference Co No. **2** slightly (Table 3).

### 1.2. Isobolographic analysis of interactions between reference Co. No. 1 and Levetiracetam in the 6 Hz seizure model

Isobolographic studies were conducted for the combined administration of reference Co. No. **1** with LEV in the 6Hz (44mA) assay. Studies were conducted according to previously described methods (Madsen et al. 2011). Initial ED₅₀ values were determined for both reference Co. No. **1** and LEV and used to calculate theoretical ED₅₀ (± standard error of the mean, SEM) values for three fixed dose ratio combinations (LEV: reference Co. No. **1**): 1:3, 1:1, and 3:1. Doses used were proportional to calculated ED₅₀ values. For example, the dose ratio used for the 1:1 paradigm was based on 0.5 x ED₅₀ for LEV and 0.5 x ED₅₀ for reference Co. No. **1**. Similarly, the 1:3 paradigm used 0.25 x ED₅₀ for LEV and 0.75 x ED₅₀ for reference Co. No. **1**. The 3:1 dose ratio used 0.75 x ED₅₀ LEV and 0.25 x ED₅₀ for reference Co. No. **1**. Experimental treatment doses (see Table 4) were based on theoretical values and adjusted according to observed effects. Experimentally determined ED₅₀ (± SEM) values for each fixed dose-ratio combination were compared to the theoretical values (t-test) for statistical purposes. The dose ratio was determined to be supra-additive (synergistic) if the experimentally-determined ED₅₀ value was significantly lower than the theoretical ED₅₀. Subsequently, the experimental combined doses were determined for the same paradigms in the 6Hz seizure test (Table 4 below). The isobolographic study with compound 1 and levetiracetam in the 6 Hz model demonstrates a significant synergistic pharmacodynamic interaction at all dose ratios evaluated and corresponds closely with reference compound 1 plasma levels. Furthermore, no motor impairment was observed at any of the dose ratios evaluated suggesting that the synergistic pharmacodynamics interaction does not produce increased motor toxicity.

### 1.3. Mouse Corneal Kindling Model and studies with reference compound 1

Mice were kindled electrically with 3 second, 3 mA, 60 Hz stimulus, twice daily using corneal electrodes until a criterion of 5 consecutive Stage 5 seizures as defined by Racine (Racine "Modification of seizure activity by electrical stimulation" II. motor seizure" Electroenceph Clin Neurophysiol 1972, 32, pp. 281-294). After the mice reached a stable kindled state, the test compound or vehicle was administered and, at the previously determined TPE, each animal was given the electrical stimulus indicated above. Following stimulation, the animals were observed for the presence or absence of the seizure activity scored on the Racine scale (0 - 5) with 5 representing the highest stage rearing and falling. One dose of LEV and two doses of reference Co. No. **1** were tested individually and in combination against corneal kindled seizures. Combination of reference compound 1 with levetiracetam in this model suggests a positive pharmacodynamics interaction (Table 5 below).

A summary of the data for the compounds tested alone is presented in Table 1 and additional results of studies performed according to example 1 are listed in Tables 2-5 below.

**Table 1: Summary of the acute anticonvulsant data in the 6Hz model at 32 mA and 44 mA for the mGluR2 PAM compounds 1 (reference), 2 (reference), 11 (reference), 2-a (reference), 25-a (reference), 6-b and reference compound LY-404039 following s.c. administration (except compound 6-b, which was tested p.o.). TPE means time of peak effect, CI means confidence interval, s.c. means subcutaneous, p.o. means orally, n.t. means not tested. TPE was determined in 32 mA 6Hz test. Effects are generally observed at doses that do not produce impairment in rotarod test. For reference compounds 11 and 2-a the individual values of repeat experiments are provided. For reference compound 25a, both 0.25 and 1h time points were used for 6Hz (44mA) studies.**

| **Co. No.** | **TPE (h)** | **ED₅₀ (95% CI) mg/kg, s.c.** | | **Seizure score (dose)** |
|---|---|---|---|---|
| | | **32 mA** | **44 mA** | **Corneal kindling** |
| **Reference compound 11** | 0.5 | 4.77 (3.54 - 6.76) 9.41 (1.53-15.1) | 31.5 (15.1 - 47.3) | 2.8 (100 mg/kg) |
| **Reference compound 2** | 0.25 | 3.83 (1.62-6.71) | 5.89 (3.89 - 8.45) | 3.4 (40 mg/kg) |
| **Reference compound 1** | 0.5 | 2.8 (1.3 - 4.3) | 10.2 (3.1 - 12.4) | 3.7 (20 mg/kg) |
| **Reference compound 25-a** | 1 | 7.7 (2.3-18.4) | 1 hr TPE: 25.9 (15.5-33.7) 0.25 hr TPE: 29.1 (21.6-39.6) | n.t. |
| **Reference compound 2-a** | 0.5 | 44.7 (23.4-80.5) 20.8 (10.0-31.7) 12.2 (8.4-17.4) | 50% protection at 100 mg/kg 21 (17.9-27.4) | 4.4 (100 mg/kg) |
| **6-b** | 0.5 | 7.2 (4.2-11.8) | 16.1 (13.0-20.1) | n.t. |
| **Reference compound LY-404039** | 0.5 | 10.2 (3.62-12.4) | n.t. | 3.1 (100 mg/kg) |

**Table 1a: Summary of the 6Hz 32 mA TPE determination for reference Co. No. 1**

| **Dose (mg/kg, s.c.)** | **Time (h)** | **6Hz 32 mA** | **Motor impairment** |
|---|---|---|---|
| 10 | 4 | 0/4 | 0/0 |
| | 2 | 0/4 | 0/0 |
| | 1 | 3/4 | 0/0 |
| | 0.5 | 4/4 | 0/0 |
| | 0.25 | 4/4 | 0/0 |
| 5 | 0.5 | 8/12 | 0/0 |
| | 0.25 | 8/12 | 0/0 |
| 2.5 | 0.5 | 5/8 | 0/0 |
| | 0.25 | 1/8 | 0/0 |

| | | | |
|---|---|---|---|
| (number of mice protected in 6Hz or toxic on rotarod/number tested) | | | |

**Table 1b: Dose-response studies for reference Co. No. 1. The TPE for reference Co. No. 1 was previously determined to be 0.5 h (results shown above in table 1a). Several doses of reference Co. No. 1 were administered at this TPE and tested in the 6Hz assay, using both 32 and 44 mA stimulus intensities.**

| **Test** | **Dose (mg/kg, s.c.)** | **#protected/#tested** | **#rotarod motor impairment/#tested** |
|---|---|---|---|
| 6Hz 32 mA | 20 | 8/8 | 1/8 |
| | 10 | 7/8 | 0/8 |
| | 5 | 8/12 | 0/12 |
| | 2.5 | 7/16 | 0/16 |
| | 0.5 | 1/8 | 0/8 |
| **ED₅₀ (95% CI): 2.8 mg/kg (1.3 to 4.3)** | | | |
| 6Hz 44 mA | 20 | 8/8 | 1/8 |
| | 15 | 7/8 | 0/8 |
| | 10 | 4/8 | 0/8 |
| | 2.5 | 0/8 | 0/8 |
| **ED₅₀ (95% CI): 10.2 mg/kg (3.1 to 12.4)** | | | |

**Table 2: Summary of interaction of reference Co. No. 1 and reference Co. No. 2 with Levetiracetam (LEV) in the mouse 6Hz, 44 mA seizure model. Results are listed as number of mice exhibiting full protection/total number of mice tested in each dosing group (at the specified test compound or combination dosage levels).**

| | **Dose** | **Time (h)** | **# protected / # tested** | **# motortox / # tested** |
|---|---|---|---|---|
| LEV | 10 mg/kg i.p. | 1 | 1/6 | 0/6 |
| **Reference Co. No. 2 + LEV** | **3 mg/kg s.c.** | **0.25** | **5/6** | **0/6** |
| Reference Co. No. 2 | 3 mg/kg s.c. | 0.25 | 1/6 | 0/6 |
| LEV | 10 mg/kg i.p. | 1 | 1/8 | 0/8 |
| **Reference Co. No. 1 + LEV** | **2.5 mg/kg s.c.** | **0.5** | **6/8** | **0/8** |
| Reference Co. No. 1 | 2.5 mg/kg s.c. | 0.5 | 0/8 | 0/8 |

**Table 2a: Plasma and brain levels reference Co. No. 1. in combination study with Levetiracetam (LEV). BQL means below quantifiable limit.**

| **Reference Co. No. 1** | **LEV** | **Plasma (ng/ml)** | **Reference Co. No. 1** | **Plasma (ng/ml)** | **6 Hz Protection** |
|---|---|---|---|---|---|
| **6 Hz 44 mA** | 10 mg/kg | 9350 | 2.5 mg/kg | BQL | Yes |
| | | 8580 | | 244 | No |
| | | 10900 | | 314 | Yes |
| | | 10300 | | 382 | Yes |
| | | 9780 | | 416 | Yes |
| | | 9780 | | 377 | Yes |
| | | 13700 | | 2260* | No |
| | | 10100 | | 607 | Yes |
| **Mean Plasma Level** | | **10311** | | **657.1 (390)** | **6/8** |
| | | | | | |
| **Mean Plasma Levels (non-combination)** | **1/8** | 8254 | **0/8** | 438 | |

| | | | | | |
|---|---|---|---|---|---|
| Mean plasma level shown in parenthesis ( ) is calculated with a statistical outlier* removed | | | | | |

**Table 2b: Plasma and brain levels reference Co. No. 2 in combination study with Levetiracetam (LEV). AQL means above quantifiable limit.**

| **Reference Co. No. 2** | **LEV** | **Plasma (ng/ml)** | **Brain (ng/ml)** | **Reference Co. No. 2** | **Plasma (ng/ml)** | **Brain (ng/ml)** | **6 Hz Protection** |
|---|---|---|---|---|---|---|---|
| **6 Hz 44 mA** | 10 mg/kg | 6450 | 6290 | 3 mg/kg | 1830 | 1540 | Yes |
| | | 8200 | 7990 | | 386 | 1020 | Yes |
| | | 3540 | 4760 | | 4700 | 1310 | Yes |
| | | 3850 | NA | | 467 | NA | No |
| | | 7150 | 6380 | | AQL (>500) | 1120 | Yes |
| | | 3890 | 3960 | | 2080 | 1140 | Yes |
| | | | | | | | |
| **Mean Plasma / Brain Levels** | | **5513** | **5876** | | **1893** | **1226** | **5/6** |
| | | | | | | | |
| **Mean Plasma / Brain Levels (non-combination)** | **1/6** | 8750 | 5773 | **1/6** | 1295 | 1113 | |

| | | | | | | | |
|---|---|---|---|---|---|---|---|
| NA - sample not available for analysis | | | | | | | |

**Table 3 : 6 Hz seizure (44mA) model ED₅₀ determinations for reference No. Co. 2 and levetiracetam (LEV) alone and in combination. LEV at a dose of 10 mg/kg increased the potency of the reference Co. No. 2 (∼5-fold shift in ED₅₀). Reference Co. No. 2 at a dose of 3 mg/kg increased both the efficacy (to 100% protection) and potency of LEV (∼200-fold shift in ED₅₀). Figure 1 shows the dose-response for the 6 Hz 44mA ED₅₀ determinations for the Reference Co. No. 2 and LEV alone and in combination.**

| **Treatment** | **ED₅₀ (95% CI) mg/kg** | **Maximum Effect (% Protection)** |
|---|---|---|
| Ref. Co. No. **2** alone | 6.97 | 100% |
| | (5.44 - 8.30) | |
| Ref. Co. No. **2** | 1.35 | 100% |
| + LEV (10 mg/kg) | (0.8 - 1.9) | |
| LEV alone | ∼ 200 | 75% |
| LEV | 1.0 | 100% |
| + Ref. Co. No. **2** (3 mg/kg) | (0.23 - 2.24) | |

**Table 4: Results of Reference Co. No. 1 and Levetiracetam in Isobolographic Study.**

| **Group** | **LEV (mg/kg i.p.)** | **f** | **Ref. Co. No. 1 (mg/kg s.c.)** | **f** | **combine d dose (mg/kg)** | **Rotarod #motortox / # tested** | **6Hz (44mA) #protected /#tested** |
|---|---|---|---|---|---|---|---|
| 1:1 paradigm | 181 | 0.5 | 5.1 | 0.5 | 93.1 | 0/8 | 8/8 |
| | 90.5 | | 2.6 | | 46.6 | 0/8 | 6/8 |
| | 45.3 | | 1.3 | | 23.3 | 0/8 | 3/8 |
| | 22.6 | | 0.6 | | 11.6 | 0/8 | 3/8 |

| **ED₅₀ (95% CI; mg/kg): 22.2 (8.4-35.7)** | | | | | | | |
|---|---|---|---|---|---|---|---|
| 1:3 paradigm | 45.3 | .25 | 3.8 | .75 | 14.2 | 0/8 | 8/8 |
| | 22.6 | | 1.9 | | 7.1 | 0/8 | 4/8 |
| | 11.3 | | 1.0 | | 3.6 | 0/8 | 2/8 |

| **ED₅₀ (95% CI; mg/kg): 5.9 (3.5-8.7)** | | | | | | | |
|---|---|---|---|---|---|---|---|
| 3:1 paradigm | 271.5 | .75 | 2.6 | .25 | 204.3 | 0/8 | 8/8 |
| | 135.8 | | 1.3 | | 102.2 | 0/8 | 3/8 |
| | 67.9 | | 0.6 | | 51.1 | 0/8 | 3/8 |
| | 33.9 | | 0.3 | | 25.5 | 0/8 | 0/8 |
| **ED₅₀ (95% CI; mg/kg): 86.3 (56.8-131.4)** | | | | | | | |

The isobolographic analysis (Figure 2) demonstrates that the combination of reference Co. No. **1** and levetiracetam results in a significantly positive synergistic effect.

**Table 5: Results of reference Co. No. 1 and Levetiracetam combination study in the corneal kindling model in mice.**

| **Compound(s)** | **# protected/# tested** | **% Protected** | **Mean Seizure Score** |
|---|---|---|---|
| Vehicle (20% HPBCD@30', s.c.; 0.5% MC@60', i.p.) | 0/10 | 0% | 4.7 |
| LEV 3 mg/kg | 5/13 | 38% | 3.3 |
| Reference Co. **No. 1** 30 mg/kg | 3/12 | 25% | 4.0 |
| **LEV 3 mg/kg & reference Co. No. 1 30 mg/kg** | 10/10 | 100% | 0.6 |
| Reference Co. **No. 1** 20 mg/kg | 5/16 | 31% | 3.7 |
| **LEV 3 mg/kg & Reference Co. No. 1 20 mg/kg** | 7/10 | 70% | 1.9 |
| | Racine Seizure Score | 0 to 5 | |
| | | 0 = no seizure activity | |
| | | 5 = maximal seizure activity | |

### EXAMPLE 2 - STUDIES WITH REFERENCE COMPOUNDS 25-a AND 2-a

### 2.1. Combination Study with reference Co. No. 25-a and Levetiracetam

Independent dose-response studies were performed in the 6 Hz 44mA test for both compounds to determine ED₅₀ values at the TPE of 1 h i.p. for levetiracetam and 1 h s.c. for reference Co. No. **25-a.** The ED₅₀ value for reference Co. No. **25-a** was 25.9 mg/kg and for levetiracetam the value was estimated to be approximately 345 mg/kg. The dose-response for levetiracetam was repeated with co-administration of 10 mg/kg reference Co. No. **25-a** (a dose of reference Co. No. **25-a** that alone did not protect in the 6 Hz 44mA model). The co-administration of 10 mg/kg reference Co. No. **25-a** produced an ED₅₀ in the levetiracetam dose-response of 4.9 mg/kg (∼70-fold lower compared with levetiracetam alone) and importantly yielded full protection in the 6Hz 44mA seizure model. These results are suggestive of a positive pharmacodynamic interaction in the 6Hz seizure model between reference Co. No. **25-a** and levetiracetam.

**Table 6: Time-to-Peak Effect Determination for reference Co. No. 25-a in the 6Hz (32mA) Assay. Two doses were used in this study, 10 and 20 mg/kg, across several time points (0.25-4h). The compound showed the greatest degree of protection in the 6Hz assay between 0.25 and 1h, which was more evident at 20 mg/kg. Plasma levels of the compound generally corresponded to behavioural seizure protection. A TPE of 0.25h was used for 6Hz (32 mA) studies whereas both the 0.25 and 1h time points were used for 6Hz (44mA) studies.**

| s.c. means subcutaneous | | | | |
|---|---|---|---|---|
| **Dose (mg/kg, s.c.)** | **Time (h)** | **# protected/# tested** | **# rotarod motor impairment / # tested** | **Reference Co. No. 25-a mean plasma levels (ng/mL)** |
| 10 | 0.25 | 2/4 | 0/4 | 10,983 (2,477) |
| | 0.5 | 1/4 | 0/4 | 3,330 |
| | 1 | 1/4 | 1/4 | 700 |
| | 2 | 0/4 | 0/4 | 256 |
| | 4 | 0/4 | 0/4 | 40 |
| | | | | |
| 20 | 0.25 | 4/4 | 0/4 | 4,095 |
| | 0.5 | 3/4 | 1/4 | 2,800 |
| | 1 | 4/4 | 1/4 | 1,765 |
| | 2 | 1/4 | 0/4 | 618 |
| | 4 | 1/4 | 1/4 | 28 |

| | | | | |
|---|---|---|---|---|
| Mean plasma level shown in parenthesis () is calculated with a statistical outlier removed. | | | | |

**Table 7: Dose-Response Studies for reference Co. No. 25-a in the 6Hz Assay (32 mA^{a} and 44 mA^{b})**

| CI means confidence interval | | | | |
|---|---|---|---|---|
| **Test** | **Dose (mg/kg, s.c.)** | **# protected/ # tested** | **# rotarod motor impairment / # tested** | **Refrence Co. No. 25-a mean plasma levels (ng/mL)** |
| 6Hz 32 mA | 20 | 8/8 | 0/8 | 5,570 |
| | 15 | 3/8 | 0/8 | 1,201 |
| | 10 | 4/8 | 0/8 | 6,113 |
| | 5 | 4/8 | 0/8 | 2,558 |
| | 1 | 1/8 | 0/8 | 466 |
| **ED₅₀ (95% CI): 7.7 mg/kg (2.3 to 18.4)** | | | | |
| 6Hz 44 mA | 40 | 7/8 | 0/8 | 6,263 |
| | 30 | 3/8 | 0/8 | 7,220 |
| | 20 | 2/8 | 0/8 | 3,368 |
| | 10 | 0/8 | 0/8 | 4,345 (1,526) |
| | 5 | 0/8 | 1/8 | 1,428 |
| **ED₅₀ (95% CI): 29.1 mg/kg (21.6 to 39.6)** | | | | |

| | | | | |
|---|---|---|---|---|
| ^{a}The time-to-peak effect in the 6Hz 32mA assay for reference Co. No. 25-a was determined to be 0.25 h (see Table 1). ^{b}The time-to-peak effect in the 6Hz 44 mA assay for reference Co. No. 25-a was similar for 0.25 h and 1 h; results for 1h confirmed the ED₅₀ (95% CI) 25.9 (15.5 - 33.7) (see Table 1 and 6). Mean plasma level shown in parenthesis () is calculated with a statistical outlier removed. | | | | |

**Table 8: Combination Studies for reference Co. No. 25-a with Levetiracetam (LEV) in the 6Hz (44 mA) Assay.**

| **Drug** | **Dose (mg/kg, s.c.)** | **# protected / # tested** | **# rotarod motor impairment / # tested** |
|---|---|---|---|
| LEV | 200 | 2/8 | 0/8 |
| | 400 | 4/9 | 0/9 |
| | 800 | 10/12 | 0/12 |
| **ED₅₀ (95% CI): 345.4 mg/kg (211.0 to 485.3)** | | | |
| LEV + reference Co. No. **25-a** 10 mg/kg | 200 | 8/8 | 1/8 |
| | 100 | 7/8 | 2/8 |
| | 50 | 5/8 | 1/8 |
| | 10 | 4/8 | 0/8 |
| | 1 | 4/8 | 1/8 |
| **ED₅₀ (95% CI): 4.9 (0.0 - 14.2)** | | | |

| | | | |
|---|---|---|---|
| Reference Co. No. **25-a** (s.c.) 10 mg/kg tested in combination with LEV (i.p.) - Reference Co. No. **25-a** 10 mg/kg, not active when administered alone. | | | |

### 2.2. Combination Study with reference Co. No. 2-a and Levetiracetam

Dose-response studies were performed in the 6 Hz 32 mA and 44mA tests (table 9 below) and in the combination test with levetiracetam (effect of reference Co. No. 2-a on the dose-response of LEV in tables 10a and effect of LEV on the dose-response of reference Co. No. 2-a in table 10b below) in the same manner as described for the studies with reference Co. No. **25-a** and levetiracetam above.

**Table 9: Dose-Response Studies for reference Co. No. 2-a in the 6Hz Assay (32 mA and 44 mA; 0.5 h TPE). A time-to-peak effect of 0.5 h was determined in the 32 mA 6 Hz test (s.c.) and used for 6Hz (32 mA and 44 mA) studies.**

| Test | Dose (mg/kg, s.c.) | # protected / # tested | # rotarod motor impairment / # tested |
|---|---|---|---|
| 6Hz 32 mA | 40 | 8/8 | 2/8 |
| | 20 | 6/8 | 3/8 |
| | 10 | 4/8 | 0/8 |
| | 5 | 0/8 | 0/8 |
| | 2.5 | 0/8 | 1/8 |
| **ED₅₀ (95% CI): 12.2 mg/kg (8.4 to 17.4)** | | | |
| 6Hz 44 mA | 40 | 8/8 | 4/8 |
| | 20 | 3/8 | 0/8 |
| | | 3/8 | 0/8 |
| | 15 | 2/8 | 1/8 |
| | **10** | **0/8** | **1/8** |
| | | **0/8** | **0/8** |
| **ED₅₀ (95% CI): 21.0 mg/kg (17.9 to 27.4) a TD₅₀ : > 40 mg/kg** | | | |

| | | | |
|---|---|---|---|
| ^{a}40 mg/kg - 6 out of 16 total (32 mA and 44 mA combined) with impairment. Dose selected for combination studies with LEV in 6Hz (44mA): reference Co. No. **2-a** 10 mg/kg. | | | |

**Table 10a: Combination Studies for reference Co. No. 2-a with Levetiracetam (LEV) in the 6Hz (44 mA) Assay. Combination of 10 mg/kg reference Co. No. 2-a with varying doses of levetiracetam.**

| **Drug** | **Dose (mg/kg)** | **# protected / # tested** | **# rotarod motor impairment / # tested** |
|---|---|---|---|
| LEV | 200 | 2/8 | 0/8 |
| | 400 | 4/9 | 0/9 |
| | 800 | 10/12 | 0/12 |
| **LEV ED₅₀ (95% CI): 345.4 mg/kg (211.0 to 485.3)** | | | |
| LEV + reference Co. No. **2-a** 10 mg/kg ^{a} | 200 | 6/8 | 1/8 |
| | 100 | 6/8 | 0/8 |
| | 50 | 6/8 | 0/8 |
| | 25 | 8/8 | 0/8 |
| | 12.5 | 5/8 | 0/8 |
| | 6.25 | 4/8 | 0/8 |
| | 3.125 | 3/8 | 1/8 |
| | 1.5625 | 0/8 | 0/8 |
| **LEV ED₅₀ (95% CI): 9.6 mg/kg (1.7 - 21.9)** | | | |

| | | | |
|---|---|---|---|
| ^{a}Reference Co. No. **2-a** (s.c.) 10 mg/kg tested in combination with LEV (i.p.); reference Co. No. **2-a** 10 mg/kg, not active when administered alone. Additional LEV (low-dose) control groups were tested at 25 and 6.25 mg/kg (1/8 and 0/6 protected, respectively). Vehicle-treated mice (0.5% methylcellulose i.p. (1h) / 20% HPBCD s.c. (0.5h)) showed no protection (0/8 protected). | | | |

**Table 10b: Combination Studies for reference Co. No. 2-a with Levetiracetam (LEV) in the 6Hz (44 mA) Assay. Combination of 350 mg/kg levetiracetam with varying doses of reference Co. No. 2-a.**

| **Drug** | **Dose (mg/kg)** | **# protected / # tested** | **# rotarod motor impairment / # tested** |
|---|---|---|---|
| **a LEV (alone)** | 350 | 3/8 | 0/8 |
| **LEV 350 mg/kg + reference b Co. No. 2-a** | 20 | 8/8 | 2/8 |
| | 10 | 7/8 | 1/8 |
| | 5 | 7/8 | 1/8 |
| | 2.5 | 5/8 | 0/8 |
| | 1.25 | 4/8 | 0/8 |
| **previous reference Co. No. 2-a ED₅₀ (95% CI): 21.0 mg/kg (17.9 to 27.4) LEV combination reference Co. No. 2-a ED₅₀ (95% CI): 1.5 mg/kg (0.1 - 2.7) ∼14-fold shift in potency** | | | |

| | | | |
|---|---|---|---|
| ^{a}LEV ED₅₀ (presented separately) previously determined in 6Hz (44 mA): 345 mg/kg. ^{b}Reference Co. No. **2-a** (s.c.) 10 mg/kg tested in combination with LEV (i.p.); reference Co. No. **2-a** 10 mg/kg, not active when administered alone. Additional LEV (low-dose) control groups were tested at 25 and 6.25 mg/kg (1/8 and 0/6 protected, respectively). Vehicle-treated mice (0.5% methylcellulose i.p. (1h) / 20% HPBCD s.c. (0.5h) showed no protection (0/8 protected). | | | |

At a dose of 10 mg/kg s.c., reference Co. No. **2-a** increases the potency of LEV, leading to an approximate 35-fold shift in the ED₅₀. This suggests a positive pharmacodynamic relationship (Table 10a). At a dose of 350 mg/kg i.p., LEV increases the potency of reference Co. No. 2-a, leading to an approximate 14-fold shift in the ED₅₀. This suggests a positive pharmacodynamics relationship (Table 10b).

### EXAMPLE 3 - STUDIES WITH COMPOUND 6-b

### 3.1. Combination Study with Co. No. 6-b and Levetiracetam

Independent dose-response studies were performed in the 6 Hz 44mA test for both compounds to determine ED₅₀ values at the TPE of 1 h i.p. for levetiracetam and 0.5 h p.o. for Co. No. **6-b**. The ED₅₀ value for Co. No. **6-b** was 16.1 mg/kg and for levetiracetam the value was estimated to be approximately 345 mg/kg. The dose-response for levetiracetam was repeated with co-administration of 10 mg/kg Co. No. **6-b** (a dose of Co. No. **6-b** that alone did not protect in the 6 Hz 44mA model). The co-administration of 10 mg/kg Co. No. **6-b** produced an ED₅₀ in the levetiracetam dose-response of 2.4 mg/kg (∼100-fold lower compared with levetiracetam alone) and importantly yielded full protection in the 6Hz 44mA seizure model. These results are suggestive of a positive pharmacodynamic interaction in the 6Hz seizure model between Co. No. **6-b** and levetiracetam.

The results of the studies performed with compound 6-b are listed in Tables 11-13 below.

**Table 11: Time-to-Peak Effect Determination for Co. No. 6-b (p.o.) in the 6HZ (32mA) Assay.**

| **Dose (mg/kg, p.o.)** | **Time (h)** | **# protected / # tested** | **# rotarod motor impairment / # tested** |
|---|---|---|---|
| **10** | 0.25 | 1/4 | 0/4 |
| | 0.5 | 3/4 | 0/4 |
| | 1 | 0/4 | 0/4 |
| | 2 | 1/4 | 0/4 |
| | 4 | 0/4 | 0/4 |
| | | | |
| **20** | 0.25 | 4/4 | 0/4 |
| | 0.5 | 3/4 | 0/4 |
| | 1 | 4/4 | 0/4 |
| | 2 | 0/4 | 0/4 |
| | 4 | 1/4 | 0/4 |

| | | | |
|---|---|---|---|
| TPE determined to be 0.5h. | | | |

**Table 12: Dose-Response Study for Co. No. 6-b in the 6Hz Assay (32mA and 44mA; 0.5h TPE).**

| **Test** | **Dose (mg/kg, p.o.)** | **# protected / # tested** | **# rotarod motor impairment / # tested** |
|---|---|---|---|
| 6Hz 32mA | 20 | 7/8 | 0/8 |
| | 10 | 6/8 | 0/8 |
| | 5 | 2/8 | 0/8 |
| | 2.5 | 1/8 | 0/8 |
| **ED₅₀ (95% CI): 7.2 mg/kg (4.2 to 11.8)** | | | |
| 6Hz 44mA | 40 | 8/8 | 0/8 |
| | 20 | 6/8 | 0/8 |
| | 15 | 4/8 | 0/8 |
| | 10 | 0/8 | 0/8 |
| **ED₅₀ (95% CI): 16.1 mg/kg (13.0 to 20.1)** | | | |

**Table 13: Combination Studies for Co. No. 6-b with LEV in the 6Hz Assay (44mA).**

| **Drug** | **Dose (mg/kg)** | **# protected / # tested** | **# rotarod motor impairment / # tested** |
|---|---|---|---|
| LEV | 200 | 2/8 | 0/8 |
| | 400 | 4/9 | 0/9 |
| | 800 | 10/12 | 0/12 |
| **ED₅₀ (95% CI): 345.4 mg/kg (211.0 to 485.3)** | | | |
| LEV + Co. No. **6-b** 10 mg/kg | 200 | 8/8 | 0/8 |
| | 100 | 8/8 | 0/8 |
| | 50 | 5/8 | 0/8 |
| | 10 | 5/8 | 0/8 |
| | 1 | 5/8 | 0/8 |
| **ED₅₀ (95% CI): 2.4 (0.0 - 6.4)** | | | |

| | | | |
|---|---|---|---|
| Co. No. **6-b** (p.o.) 10 mg/kg tested in combination with LEV (i.p.) Co. No. **6-b** 10 mg/kg, not active when administered alone | | | |

### EXAMPLE 4 - STUDIES WITH REFERENCE COMPOUND LY404039

### 3.1. Combination Study with reference LY404039 and Levetiracetam

Reference compound LY-404039 was tested alone and in combination with levetiracetam according to the procedures already described hereinabove. The results of the studies performed with reference compound LY-404039 are listed in tables 14-15.

**Table 14: Dose-Response Studies for reference compound LY404039 in the 6Hz Assay (32 mA and 44 mA). A time-to-peak effect of 0.5 h was determined in the 32 mA 6 Hz test (s.c.) and used for 6Hz (32 mA and 44 mA) studies.**

| **Test** | **Dose (mg/kg, s.c.)** | **# protected / # tested** | **# rotarod motor impairment / # tested** |
|---|---|---|---|
| 6Hz 32mA | 40 | 8/8 | 1/8 |
| | 20 | 6/8 | 1/8 |
| | 10 | 5/8 | 0/8 |
| | 5 | 1/16 | 1/16 |
| **ED₅₀ (95% CI): 10.9 mg/kg (7.8 to 15.9)** | | | |
| 6Hz 44mA | 40 | 7/8 | 2/8 |
| | 20 | 7/8 | 1/8 |
| | 10 | 3/8 | 1/8 |
| | **5** | **0/16** | **0/16** |
| **ED₅₀ (95% CI): 14.1 mg/kg (10.0 to 20.6)** | | | |
| **a TD₅₀ : > 40 mg/kg** | | | |

| | | | |
|---|---|---|---|
| ^{a}40 mg/kg - 3 out of 16 total (32 mA and 44 mA combined) with impairment. Note: no activity observed following vehicle administration in 32 or 44 mA. Dose selected for combination studies with LEV in 6Hz (44 mA): reference compound LY404039 5 mg/kg. | | | |

**Table 15: Combination Studies for reference compound LY404039 with Levetiracetam (LEV) in the 6Hz (44 mA) Assay.**

| **Drug** | **Dose (mg/kg)** | **# protected / # tested** | **# rotarod motor impairment / # tested** |
|---|---|---|---|
| **LEV^{a}** | 200 | 2/8 | 0/8 |
| | 400 | 4/9 | 0/9 |
| | 800 | 10/12 | 0/12 |
| **LEV ED₅₀ (95% CI): 345.4 mg/kg (211.0 to 485.3)** | | | |
| **LEV + ref. compound LY404039 5 b mg/kg** | 200 | 8/8 | 0/8 |
| | 50 | 6/8 | 1/8 |
| | 20 | 6/8 | 2/8 |
| | 5 | 2/8 | 1/8 |
| **LEV ED₅₀ (95% CI): 12.8 mg/kg (2.5 - 25.2)** | | | |

| | | | |
|---|---|---|---|
| ^{a}LEV alone shown previously, confirmation doses performed in combination with reference Co. No. 2-a (see previous table above). ^{b}Reference compound LY404039 (s.c.) 5 mg/kg tested in combination with LEV (i.p.); reference compound LY404039 5 mg/kg was not active when administered alone. Additional LEV (low-dose) control groups were tested at 25 and 6.25 mg/kg (1/8 and 0/6 protected, respectively). Vehicle-treated mice (10% sterile water - NaCl; s.c., 0.5 h TPE and 0.5% MC, i.p., 1h TPE) showed no protection or rotarod impairment. | | | |

At a dose of 5 mg/kg reference compound LY404039 increases the potency of LEV, leading to an approximate 27-fold shift in the ED₅₀. This suggests a positive pharmacodynamic relationship.

### EXAMPLE 5 - REFERENCE STUDIES WITH REFERENCE COMPOUND CAS 1092453-15-0

### 4.1. Combination Study with reference compound 2,3-dihydro-7-methyl-5-[3-(1-piperazinylmethyl)-1,2,4-oxadiazol-5-yl]-2-[[4-(trifluoromethoxy)phenyl]methyl]-1H-isoindol-1-one [CAS 1092453-15-0] (described in WO 2008150233, WO 2011084098) and Levetiracetam

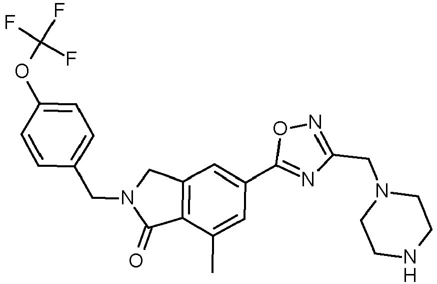

Reference compound CAS 1092453-15-0 was tested alone and in combination with levetiracetam according to the procedures already described hereinabove. The results of example 5 are listed in tables 16-17.

**Table 16: Dose-Response Studies for reference compound CAS 1092453-15-0 in the 6Hz Assay (32 mA).**

| **Dose (mg/kg, s.c.)** | **Time (h)** | **# protected / # tested** | **# rotarod motor impairment / # tested** |
|---|---|---|---|
| **20** | 0.25 | 1/4 | 0/4 |
| | 0.5 | 0/4 | 0/4 |
| | 1 | 1/4 | 0/4 |
| | 2 | 0/4 | 0/4 |
| | 4 | 0/4 | 0/4 |
| **40** | 0.25 | 1/4 | 0/4 |
| | 0.5 | 1/4 | 0/4 |
| | 1 | 1/4 | 0/4 |
| | 2 | 0/4 | 0/4 |
| | 4 | 0/4 | 0/4 |
| **80** | 0.25 | 0/4 | 0/4 |
| | 0.5 | 0/4 | 0/4 |
| | 1 | 1/4 | 1/4 |

Low activity was observed at doses and time points tested. Greatest activity at 0.25-1 h in tested doses. Combination studies were performed using 20 mg/kg, s.c, 1h TPE in the 6Hz (44 mA) assay.

**Table 17: Combination Studies for reference compound CAS 1092453-15-0 with Levetiracetam (LEV) in the 6Hz (44 mA) Assay.**

| **Drug** | **LEV Dose (mg/kg)** | **# protected / # tested** | **# rotarod motor impairment / # tested** |
|---|---|---|---|
| **LEV^{a}** | 200 | 2/8 | 0/8 |
| | 400 | 4/9 | 0/9 |
| | 800 | 10/12 | 0/12 |
| **LEV ED₅₀ (95% CI): 345.4 mg/kg (211.0 to 485.3)** | | | |
| **Reference compound [**CAS 1092453-15-0**] (20 mg/kg, alone)** | | 0/8 | 0/8 |
| **LEV + Reference compound [**CAS 1092453-b 15-0**] 20 mg/kg^{b}** | 400 | 4/8 | 0/8 |
| | 200 | 5/8 | 0/8 |
| | 50 | 3/8 | 0/8 |
| | 20 | 2/8 | 0/8 |
| | 5 | 1/8 | 1/8 |
| **LEV ED₅₀ (95% CI): 238.9 mg/kg (41.6 - above highest dose tested)** | | | |

| | | | |
|---|---|---|---|
| ^{a}Additional LEV (low-dose) control groups were tested at 25 and 6.25 mg/kg (1/8 and 0/6 protected, respectively). ^{b}Reference compound [CAS 1092453-15-0] 20 mg/kg (s.c.; 1h TPE) tested in combination with LEV (i.p.; 1h TPE); reference compound [CAS 1092453-15-0] 20 mg/kg displayed low activity when administered alone (6Hz, 32 mA), and it was not tested in 6Hz (44mA). This compound displayed an *in vitro* EC₅₀ = 562 nM (Eₘₐₓ = 197%) when tested in the GTPγS assay described hereinbefore and no occupancy was observed in ex vivo experiments in rats. Note: Vehicle-treated animals (10% HPβCD-NaCl, s.c., 1 h and 0.5% MC, i.p., 1h) showed no protection or motor impairment, N=8. | | | |

The current data set indicates that mGlu2 PAM or agonist molecules have anticonvulsant activity in the 6Hz animal model. Tested mGlu2 PAMs with EC₅₀ potencies ≤150 nM (as determined in the [³⁵S]GTPγS assay), appropriate PK parameters and brain penetration, showed activity in both the 32 and 44 mA 6Hz paradigm. Furthermore, all the tested molecules showed synergistic effects with LEV. In contrast, reference molecule CAS 1092453-15-0, which was only weakly active (EC₅₀ 562 nM) in vitro, did not show activity in either of the 6Hz tests, and also did not display synergy with LEV.

Importantly, the data indicate that, under conditions of comparable PK characteristics and appropriate brain penetration, the most potent mGlu2 PAMs, based on in vitro EC₅₀ values, also appeared most potent *in vivo,* suggesting that *in vitro* and *in vivo* potency can be linked. Moreover, synergistic effects with LEV were consistently seen with mGlu2 PAM doses similar to the ED₅₀ obtained in the 32 mA model or at least 2-fold lower as the ED₅₀ determined in the 44 mA paradigm (i.e. a dose inactive in the 44mA test when the molecules were tested alone).

Also for reference compound LY404039, the mGlu2/3 agonist, activity in both 6Hz tests was seen and synergy was seen at a dose 3-fold lower than the ED₅₀ determined in the 44 mA model, which was inactive when tested alone.

Based on the available preclinical data in the 6Hz 44mA model, it seems that combining a potent SV2A ligand and a potent mGlu2 PAM, leads to a decrease in the median effective dose or ED₅₀ of the SV2A ligand, such as LEV, between 35 and 100-fold.

Thus, while not wishing to be bound by theory, it is suggested that positive allosteric modulator of metabotropic glutamatergic receptor subtype 2 (mGluR2 PAM) compounds, in particular mGluR2 PAM compounds having an EC₅₀ potency of ≤150 nM (as determined in the [³⁵S]GTPγS assay), wherein EC₅₀ is the concentration producing half-maximal effect in a concentration-response curve obtained in the presence of EC₂₀ of glutamate, and appropriate PK parameters and brain penetration, result in a synergistic combination with an SV2A ligand, in particular levetiracetam, at non-effective doses of one or both of compound (a) and compound (b) of the combination of the invention.

Thus, in a further embodiment, the positive allosteric modulator of metabotropic glutamatergic receptor subtype 2 (mGluR2 PAM) compound of the combination of the invention as defined herein is selected from an mGluR2 PAM compound having an EC₅₀ potency of ≤150 nM (as determined in the [³⁵S]GTPγS assay), wherein EC₅₀ is the concentration producing half-maximal effect in a concentration-response curve obtained in the presence of EC₂₀ of glutamate.

### PROPHETIC EXAMPLES

### A) DOMINANT-SUBMISSIVE RELATIONS (DSR) IN RAT IN VIVO ASSAY

The DSR assay is divided into two models: Reduction of Dominant Behavior Model (RDBM) of mania and Reduction of Submissive Behavior Model (RSBM) of depression. The RDBM, wherein the dominant animals are treated with test compound, is predictive of the ability of the test compound to treat mania. The RSBM, wherein the submissive animals are treated with test compound, is predictive of the ability of the test compound to treat depression.

Male Sprague Dawley rats (140 to 160g) from Charles River Laboratories Wilmington, MA are used in this assay. Shipments of rats are received at two-week intervals. Each shipment will go through five-day quarantine, one-week acclimation period and one-week selection process, followed by five-weeks of drug or vehicle treatment to those pairs selected.

Rats will be housed four per cage. Access to food will be restricted to one hour per day after testing on Monday through Thursday. After testing on Friday, rats will have free access to food until being fasted again on Sunday. At no time will the rats be deprived of water. The food deprivation periods used will have little effect on weight gain as the average weight of rats will be about 300g by the end of the study. At the conclusion of experiment rats will be sacrificed by decapitation, the trunk blood and brains will be collected for in vitro experiments and drug concentration measurements.

The basic testing apparatus consisted of two chambers connected with a tunnel only large enough to allow one rat to pass through at a time. On the floor, at the mid-point of the tunnel will be a container of sweetened milk. This basic apparatus will be replicated, so that a total of four pairs of rats can be video tracked simultaneously. The camera can distinguish rats marked by different colors. Thus, the rats' heads will be colored for the purpose of video tracking, red in one cage and yellow in the other cage. Only one animal at a time can have comfortable access to the feeder, but both animals can drink milk during the five-minute daily session. During the five-minute daily sessions, time spent in the feeder zone by each rat will be recorded by the video tracking software and saved into a text file.

The test will begin with a random assignment of rats into pairs. Each member of a pair will be placed in an opposite chamber of the testing apparatus. The time spent in the feeder zone by each animal will be recorded. During the first week (five days) of testing the animals habituate to the new environment. Dominance will be assigned to the animal with the highest score during the second week of testing if three criteria are achieved. First, there must be a significant difference (two-tailed t-test, P<0.05) between the average daily drinking scores of both animals. Second, the dominant animal score must be at least 25% greater than the submissive animal's score. Finally, there must be no "reversals" during the pair selection week where the putative submissive rat out-scored its dominant partner on isolated occasions. Ideally there will be minimal reversals during the acclimation week as well. Only animal pairs that achieve these criteria will be continued in the study.

Significant differences between time spent on the feeder by dominant and submissive rats will be determined by ANOVA using GraphPad Prism software (GraphPad Software, Inc. San Diego, CA) followed by a two-tailed t-test (P<0.05). Comparisons will be made between treatment groups using normalized dominance level values in paired animals. The dominance level is a value that measures social relation between paired subjects. Dominance level (DL) = FTD-FTS where FTD is the feeder time of dominant rats and FTS is the feeder time of submissive rats. The normalization will be conducted according to the formula: $Dominance Level \left(week n in %\right) = \left(Dominance Level\left(week n\right)\right) / \left(Dominance Level\left(week 2\right)\right)$

The statistical significance of the difference in dominance level between the control group (pairs of rats where both dominant and submissive animals will be treated with vehicle) and the treatment group (submissive rats will be treated with drug and dominant rats with vehicle) will be determined by ANOVA, followed by a t-test. The activity onset time value at 50% of response (AOT-50) and the minimum and maximum response to drug will be calculated based on the reduction of the dominance level value using non-linear regression analysis (GraphPad Software, Inc., San Diego, CA). The normalized DL values will be used for this calculation, where DL values for treatment weeks will be normalized as a percent of the second week (pretreatment) value of that pair according the above formula. In these settings the minimum of the response (DL) determines drug positive activity, corresponding to efficacy, since DL values will be reduced if the response to a drug is positive. In the case of the negative response to a drug (worsening of symptoms) DL values will be increased. If the drug does not have such activity the maximum of the response will not exceed 100%. Any maximal DL value significantly higher than control value (about 100%) indicates drug negative activity.

Levetiracetam and mGluR2 PAM compound (e.g. compound 6-b) will be evaluated in the rat RDBM according to the procedure described in more detail below.

Groups of dominant rats will be treated p.o. QD with levetiracetam 10 mg/kg and mGluR2 PAM compound at various concentrations from approximately 0.05 mg/kg (n≥3), at 0.5 mg/kg (n≥3), at 2.5 mg/kg (n≥3), at 5.0 mg/kg (n≥3) and at 50.0 mg/kg (n≥3). A vehicle control group of dominant rats will be treated with 0.5% methylcellulose (n≥3) and a second control group of dominant rats will be treated i.p. QD with sodium valproate at 30 mg/kg (n≥6 from 2 studies of n≥3 each).

All treatments will be administered approximately 1 hour prior to testing. All treatments will be started on Saturday after the second testing week (selection week). The levetiracetam and mGluR2 PAM compound will be administered orally (p.o.).

When dominant animals are treated with levetiracetam 10mg/kg and mGluR2 PAM compound the difference between dominant and submissive rats will be lost after the first or second week of treatment depending on the dosage. Similarly, when dominant animals are treated with sodium valproate, the difference between dominant and submissive rats will also be lost after first week of treatment. The permissiveness of the dominant rats treated with levetiracetam and mGluR2 PAM compound or sodium valproate may be observed to increase. Thus the treated dominant rats will permit their submissive partners to increase their time on the feeder.

To compare different drug and dose effects the data will be normalized to the initial control week values. The strongest effect of levetiracetam and mGluR2 PAM compound combination will be observed where there is a significant difference in dominance level (DL) values between vehicle and combination treated rats starting in the second week and continuing through the treatment duration of 5 weeks. In comparison, animals (30 mg/kg) that will be treated with sodium valproate will consistently show a decreased dominance level after the second week of treatment with the effect increasing in the following weeks.

To estimate activity onset time (AOT), daily average values for feeder time of dominant and submissive animal pairs will be plotted and significant differences between these two groups will be calculated using the two-tail t-test.

To compare activity onset time (AOT) between different treatments the activity onset time will be estimated from the non-linear regression fit. The non-linear regression model will fit for each drug, combination and dose normalized daily DL values. Effects of levetiracetam and mGluR2 PAM compound in the RDBM are expected to be dose dependent.

In this assay, the combination of levetiracetam and mGlu2 PAM compounds is expected to reduce dominant behavior indicating that the combination is active as an anti-manic.

### B) ORAL TABLETS

As a specific example of an oral composition, 100 mg of a mGluR2 PAM compound is formulated with sufficiently finely divided lactose to provide a total amount of 580 to 590 mg to fill a size O hard gel capsule.

## Claims

1. A combination comprising
(a) a synaptic vesicle protein 2A ("SV2A") ligand selected from the group consisting of levetiracetam, brivaracetam and seletracetam; and
(b) a positive allosteric modulator ("PAM") of metabotropic glutamatergic receptor subtype 2 ("mGluR2") compound selected from 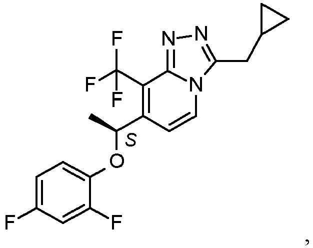 or a pharmaceutically acceptable salt or a solvate thereof.

2. The combination of claim 1, wherein the positive allosteric modulator of metabotropic glutamatergic receptor subtype 2 is 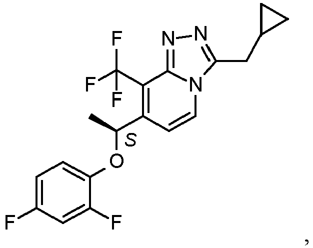 or a hydrochloride salt thereof.

3. The combination according to claim 1 or 2, wherein the SV2A ligand is levetiracetam.

4. The combination according to claim 1 or 2, wherein the SV2A ligand is brivaracetam.

5. A pharmaceutical composition comprising a combination as claimed in any one of claims 1 to 4, and a pharmaceutically acceptable carrier.

6. The pharmaceutical composition according to claim 5, formulated as a combined pharmaceutical composition.

7. The pharmaceutical composition according to claim 5, formulated in separate pharmaceutical compositions.

8. A process for the preparation of the pharmaceutical composition according to claim 5 or 6, wherein the combination as claimed in any one of claims 1 to 4 is intimately admixed with a pharmaceutically acceptable carrier.

9. A product comprising the combination as defined in any one of claims 1 to 4, as a combined preparation for simultaneous, separate or sequential use in the treatment or prevention of epilepsy and related disorders; neuropathic pain; migraine or resistant headache; bipolar and related disorders.

10. A combination as defined in any one of claims 1 to 4, or a pharmaceutical composition as defined in any one of claims 5 to 7, for use as a medicament.

11. A combination as claimed in any one of claims 1 to 4 for use in the treatment or prevention of epilepsy; neuropathic pain; migraine or resistant headache; and bipolar and related disorders.

12. The combination for use according to claim 11, in the treatment or prevention of epilepsy.

13. The combination for use according to claim 12, wherein epilepsy is treatment resistant epilepsy.

14. The combination for use according to claim 12, wherein epilepsy is epilepsy with partial-onset seizures with or without generalization.

15. The combination for use according to claim 12, wherein epilepsy is epilepsy with generalized seizures.

16. The combination for use according to claim 12, wherein epilepsy is epilepsy with primary generalized tonic-clonic seizures.

17. A combination as claimed in any one of claims 1 to 4, or a pharmaceutical composition as defined in any one of claims 5 to 7, for use in the prevention of epileptogenesis.

18. A pharmaceutical product or a commercial package comprising a combination according to any one of claims 1 to 4 together with instructions, for simultaneous, separate or sequential use thereof in the treatment or prevention of epilepsy; neuropathic pain; migraine or resistant headache; and bipolar and related disorders.

## Patentansprüche

1. Kombination, umfassend
(a) einen Liganden des synaptischen Vesikelproteins 2A ("SV2A") aus der Gruppe bestehend aus Levetiracetam, Brivaracetam und Selectracetam und
(b) einen positiven allosterischen Modulator ("PAM") des metabotropen glutamatergen Rezeptors Subtyp 2 ("mGluR2"), ausgewählt aus 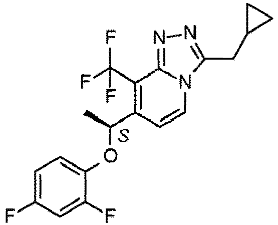 oder einem pharmazeutisch unbedenklichen Salz oder Solvat davon.

2. Kombination nach Anspruch 1, wobei es sich bei dem positiven allosterischen Modulator des metabotropen glutamatergen Rezeptors Subtyp 2 um 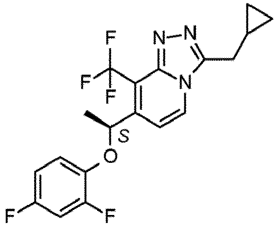 oder ein Hydrochloridsalz davon handelt.

3. Kombination nach Anspruch 1 oder 2, wobei es sich bei dem SV2A-Liganden um Levetiracetam handelt.

4. Kombination nach Anspruch 1 oder 2, wobei es sich bei dem SV2A-Liganden um Brivaracetam handelt.

5. Pharmazeutische Zusammensetzung, umfassend eine Kombination nach einem der Ansprüche 1 bis 4 und einen pharmazeutisch unbedenklichen Träger.

6. Pharmazeutische Zusammensetzung nach Anspruch 5, die als kombinierte pharmazeutische Zusammensetzung formuliert ist.

7. Pharmazeutische Zusammensetzung nach Anspruch 5, die in separaten pharmazeutischen Zusammensetzungen formuliert ist.

8. Verfahren zur Herstellung der pharmazeutischen Zusammensetzung nach Anspruch 5 oder 6, bei dem man die Kombination gemäß einem der Ansprüche 1 bis 4 innig mit einem pharmazeutisch undebenklichen Träger mischt.

9. Produkt, umfassend die Kombination gemäß einem der Ansprüche 1 bis 4 als Kombinationspräparat für die gleichzeitige, separate oder aufeinanderfolgende Verwendung bei der Behandlung oder Prävention von Epilepsie und verwandten Störungen, neuropathischen Schmerzen, Migräne oder resistenten Kopfschmerzen und bipolaren und verwandten Störungen.

10. Kombination gemäß einem der Ansprüche 1 bis 4 oder pharmazeutische Zusammensetzung gemäß einem der Ansprüche 5 bis 7 zur Verwendung als Medikament.

11. Kombination nach einem der Ansprüche 1 bis 4 zur Verwendung bei der Behandlung oder Prävention von Epilepsie, neuropathischen Schmerzen, Migräne oder resistenten Kopfschmerzen und bipolaren und verwandten Störungen.

12. Kombination zur Verwendung nach Anspruch 11 bei der Behandlung oder Prävention von Epilepsie.

13. Kombination zur Verwendung nach Anspruch 12, wobei es sich bei der Epilepsie um behandlungsresistente Epilepsie handelt.

14. Kombination zur Verwendung nach Anspruch 12, wobei es sich bei der Epilepsie um Epilepsie mit partiellen Anfällen mit oder ohne Generalisierung handelt.

15. Kombination zur Verwendung nach Anspruch 12, wobei es sich bei der Epilepsie um Epilepsie mit generalisierten Anfällen handelt.

16. Kombination zur Verwendung nach Anspruch 12, wobei es sich bei der Epilepsie um Epilepsie mit primär generalisierten tonisch-klonischen Anfällen handelt.

17. Kombination nach einem der Ansprüche 1 bis 4 oder pharmazeutische Zusammensetzung gemäß einem der Ansprüche 5 bis 7 zur Verwendung bei der Prävention von Epileptogenese.

18. Pharmazeutisches Produkt oder kommerzielle Packung, umfassend eine Kombination nach einem der Ansprüche 1 bis 4 zusammen mit Anweisungen zur gleichzeitigen, separaten oder aufeinanderfolgenden Verwendung bei der Behandlung oder Prävention von Epilepsie, neuropathischen Schmerzen, Migräne oder resistenten Kopfschmerzen und bipolaren und verwandten Störungen.

## Revendications

1. Combinaison comprenant
(a) un ligand de protéine de vésicule synaptique 2A (« SV2A ») choisi dans le groupe constitué par le lévétiracétam, le brivaracétam et le sélétracétam ; et
(b) un composé modulateur allostérique positif (« PAM ») du récepteur glutamatergique métabotropique de sous-type 2 (« mGluR2 ») choisi parmi 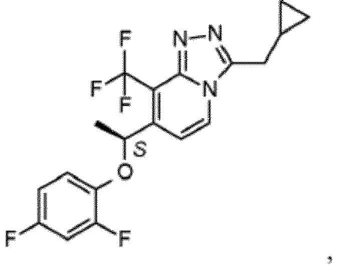 ou un sel pharmaceutiquement acceptable ou un solvate correspondant.

2. Combinaison selon la revendication 1, le modulateur allostérique positif de récepteur glutamatergique métabotropique de sous-type 2 étant 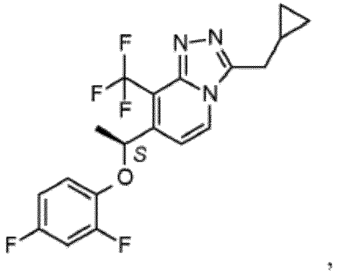 ou un sel de chlorhydrate correspondant.

3. Combinaison selon la revendication 1 ou 2, le ligand SV2A étant le lévétiracétam.

4. Combinaison selon la revendication 1 ou 2, le ligand SV2A étant le brivaracétam.

5. Composition pharmaceutique comprenant une combinaison telle que revendiquée dans l'une quelconque des revendications 1 à 4, et un support pharmaceutiquement acceptable.

6. Composition pharmaceutique selon la revendication 5, formulée en tant qu'une composition pharmaceutique combinée.

7. Composition pharmaceutique selon la revendication 5, formulée en compositions pharmaceutiques séparées.

8. Procédé pour la préparation de la composition pharmaceutique selon la revendication 5 ou 6, la combinaison telle que revendiquée dans l'une quelconque des revendications 1 à 4 étant mélangée intimement avec un support pharmaceutiquement acceptable.

9. Produit comprenant la combinaison telle que définie dans l'une quelconque des revendications 1 à 4, en tant qu'une préparation combinée pour une utilisation simultanée, séparée ou séquentielle dans le traitement ou la prévention de l'épilepsie et de troubles apparentés ; d'une douleur neuropathique ; d'une migraine ou d'un mal de tête résistant ; de troubles bipolaires et apparentés.

10. Combinaison telle que définie dans l'une quelconque des revendications 1 à 4, ou composition pharmaceutique telle que définie dans l'une quelconque des revendications 5 à 7, pour une utilisation en tant que médicament.

11. Combinaison telle que revendiquée dans l'une quelconque des revendications 1 à 4 pour une utilisation dans le traitement ou la prévention de l'épilepsie ; d'une douleur neuropathique ; d'une migraine ou d'un mal de tête résistant ; et de troubles bipolaires et apparentés.

12. Combinaison pour une utilisation selon la revendication 11, dans le traitement ou la prévention de l'épilepsie.

13. Combinaison pour une utilisation selon la revendication 12, l'épilepsie étant l'épilepsie résistante au traitement.

14. Combinaison pour une utilisation selon la revendication 12, l'épilepsie étant l'épilepsie avec des crises partielles avec ou sans généralisation.

15. Combinaison pour une utilisation selon la revendication 12, l'épilepsie étant l'épilepsie avec des crises généralisées.

16. Combinaison pour une utilisation selon la revendication 12, l'épilepsie étant l'épilepsie avec des crises tonicocloniques généralisées primaires.

17. Combinaison telle que revendiquée dans l'une quelconque des revendications 1 à 4, ou composition pharmaceutique telle que définie dans l'une quelconque des revendications 5 à 7, pour une utilisation dans la prévention de l'épileptogénèse.

18. Produit pharmaceutique ou emballage commercial comprenant une combinaison selon l'une quelconque des revendications 1 à 4 conjointement avec des instructions, pour une utilisation simultanée, séparée ou séquentielle de celle-ci dans le traitement ou la prévention de l'épilepsie ; d'une douleur neuropathique ; d'une migraine ou d'un mal de tête résistant ; et de troubles bipolaires et apparentés.
